# EUROPEAN PATENT APPLICATION

(11) **EP 4 670 728 A1**
(43) Date of publication of application: **31.12.2025**
(21) Application number: 24844242.8
(22) Date of filing: 18.03.2024
(51) Int. Cl.: A61K 35/76, A61K 45/06, A61P 35/00

(54) **METHOD FOR TREATMENT OF TUMOR BY USING RECOMBINANT ONCOLYTIC VIRUS IN COMBINATION WITH SMALL-MOLECULE ANTICANCER DRUG**

(30) Priority: 26.07.2023 CN 202310921469
(71) Applicant: Joint Biosciences (SH) Ltd., Pudong New Area Shanghai 201318 (CN)
(72) Inventor: ZHOU, Guoqing, Shanghai 201318 (CN); ZHANG, Fan, Shanghai 201318 (CN); MA, Liang, Shanghai 201318 (CN); TIAN, Ting, Shanghai 201318 (CN); CHENG, Longxin, Shanghai 201318 (CN); MA, Qibin, Shanghai 201318 (CN); MAO, Liying, Shanghai 201318 (CN)
(74) Representative: Dr. Gassner & Partner mbB
(86) International application number: PCT/CN2024/082163
(87) International publication number: WO 2025/020545

(57) **Abstract**

The present application relates to the technical field of biomedicine, and in particular to a method for treatment of a tumor by using a recombinant oncolytic virus in combination with a small-molecule anticancer drug. Specifically, the method comprises the following steps: treating a tumor by using a recombinant oncolytic virus in combination with a small-molecule anticancer drug, wherein the small-molecule anticancer drug includes a small-molecule anticancer drug targeting ALK, a small-molecule anticancer drug targeting BTK, a small-molecule anticancer drug targeting EGFR, a small-molecule anticancer drug targeting FGFR, a small-molecule anticancer drug targeting HER2, a small-molecule anticancer drug targeting Parp, a small-molecule anticancer drug targeting PI3K, a small-molecule anticancer drug targeting VEGFR, a small-molecule anticancer drug targeting CDK4/6, and a small-molecule anticancer drug targeting KRAS; and the recombinant oncolytic virus comprises an M protein, a G protein, an N protein, a P protein, and an L protein after site-directed mutagenesis. According to the present application, the recombinant oncolytic virus and the small-molecule anticancer drug are used in combination to attack and kill tumor cells, thereby achieving the synergistic efficacy.

## Description

### TECHNICAL FIELD

The present application relates to the technical field of biomedicine and, in particular, to a method for treatment of a tumor by using a recombinant oncolytic virus in combination with a small-molecule anticancer drug.

### BACKGROUND

The oncolytic viruses are a type of tumor-killing viruses with replication ability and now are accepted by the public as an important branch of tumor immunotherapy. Oncolytic viruses can specifically target and infect tumor cells, for example, by taking advantage of the inactivation or defects of oncolytic virus suppressor genes in tumor cells to selectively infect tumor cells. After oncolytic viruses infect tumor cells, they will replicate in large quantities in tumor cells and eventually destroy tumor cells, thereby killing tumor cells. Moreover, oncolytic viruses can also provide immune stimulation signals necessary to enhance the host's own anti-cancer response, thereby attracting more immune cells to continue to kill residual tumor cells.

In spite of a good application prospect of oncolytic viruses in tumor immunotherapy, wild-type oncolytic viruses often cause problems, for example, damage and dysfunction of tissues and organs. In the process of using wild-type viruses to infect tumor cells, there is still a great risk of pathogenicity. In view of this, in order to further promote the clinical application of oncolytic viruses, it is necessary to modify the wild-type oncolytic viruses to obtain attenuated oncolytic viruses. The attenuated oncolytic viruses are used in clinical practice to reduce the pathogenic risk of oncolytic viruses and increase the safety of oncolytic viruses.

However, in the process of modifying oncolytic viruses, if only the wild-type oncolytic viruses are randomly genetically modified, their cytotoxicity can be reduced, but the modified oncolytic viruses may have a poor cure rate, or even be unable to be packaged, which is not conducive to the promotion of the clinical application of oncolytic viruses.

With the development of modern molecular biology and the application of computer-aided drug design, structural biology, combinatorial chemistry and other advanced technologies, small-molecule anticancer drugs have entered a rapid development stage. To date, FDA (United States Food and Drug Administration) and NMPA (National Medical Products Administration) have approved 89 small-molecule anticancer drugs for various cancers. Thousands of targeted drugs are undergoing clinical trials for cancer treatment. A large number of promising drugs have entered phase III trials. It is predicted that the global anti-cancer drug market will reach US $200 billion in 2021, of which targeted drugs are the "main force". Despite significant progress, small-molecule anticancer drugs still face a number of challenges.

The first major challenge is drug resistance. Almost all targeted anticancer drugs encounter the problem of drug resistance after a period of clinical use. Drug resistance is associated with many mechanisms, including gene mutation, amplification, tumor stem cells, efflux transporters, dysregulation of apoptosis, and autophagy. Gene mutation is the main cause of anti-tumor drug resistance. Inefficiency is another major challenge in targeted anticancer drugs. Targeted anticancer drugs are effective for only a limited number of patients. For example, less than 20% of patients with NSCLC are sensitive to EGFR inhibitors such as Gefitinib and Erlotinib.

### SUMMARY

In order to further improve the curative effect of tumor cells, the present application provides a method for treatment of a tumor by using a recombinant oncolytic virus in combination with a small-molecule anticancer drug.

The oncolytic viruses are a type of tumor-killing viruses with replication ability and now are accepted by the public as an important branch of tumor immunotherapy. Oncolytic viruses can specifically target and infect tumor cells, for example, by taking advantage of the inactivation or defects of oncolytic virus suppressor genes in tumor cells to selectively infect tumor cells. After oncolytic viruses infect tumor cells, they will replicate in large quantities in tumor cells and eventually destroy tumor cells, thereby killing tumor cells. Moreover, oncolytic viruses can also provide immune stimulation signals necessary to enhance the host's own anti-cancer response, thereby attracting more immune cells to continue to kill residual tumor cells. Therefore, oncolytic viruses have the ability to break the microenvironment of tumor tissues and turn "cold tumors" into "hot tumors".

In the meanwhile, small-molecule anticancer drugs have entered a rapid development stage, and thousands of targeted drugs are undergoing clinical trials for cancer treatment. However, despite significant progress, small-molecule anticancer drugs still face challenges such as drug resistance and inefficiency. According to the treatment method of the present application, the recombinant oncolytic virus and the small-molecule anticancer drug are used in combination to attack and kill tumor cells, thereby achieving a synergistic efficacy.

Further, in order to improve the curative effect, cytokines may further be inserted and expressed in the oncolytic virus. Under the synergistic effect of three anti-tumor mechanisms: oncolytic virus, cytokine and small-molecule anticancer drug, a greater anti-tumor effect is achieved.

The present application provides a method for treatment of a tumor by using a recombinant oncolytic virus in combination with a small-molecule anticancer drug, adopting the following technical solutions:

A method for treatment of a tumor by using a recombinant oncolytic virus in combination with a small-molecule anticancer drug, wherein the small-molecule anticancer drug and the recombinant oncolytic virus are used in combination to treat the tumor;
the small-molecule anticancer drug includes a small-molecule anticancer drug targeting ALK, a small-molecule anticancer drug targeting BTK, a small-molecule anticancer drug targeting EGFR, a small-molecule anticancer drug targeting FGFR, a small-molecule anticancer drug targeting HER2, a small-molecule anticancer drug targeting Parp, a small-molecule anticancer drug targeting PI3K, a small-molecule anticancer drug targeting VEGFR, a small-molecule anticancer drug targeting CDK4/6, and a small-molecule anticancer drug targeting KRAS;
the recombinant oncolytic virus comprises an M protein, a G protein, an N protein, a P protein, and an L protein.

Compared to an amino acid sequence shown in SEQ ID NO 1, the M protein includes any one or more site mutations of M51R, V221F, and S226R; or the M protein includes any one or more site mutations of N32S, N49D, M51R, H54Y, V221F, V225I, and S226R; or the M protein includes any one or more site mutations of N32S, N49D, M51R, H54Y, knockouting of bases encoding leucine at position 111, V221F, V225I, and S226R; or the M protein includes any one or more site mutations of N32S, N49D, M51R, H54Y, L111A, V221F, V225I, and S226R; or the M protein includes any one or more site mutations of G21E, N32S, N49D, M51R, H54Y, V221F, V225I, and S226R; or the M protein includes any one or more site mutations of G21E, N32S, M33A, N49D, M51R, H54Y, V221F, V225I, and S226R; or the M protein includes any one or more site mutations of G21E, N32S, M33A, N49D, M51R, H54Y, A133T, V221F, V225I, and S226R; or the M protein includes any one or more site mutations of N32S, M33A, N49D, M51R, H54Y, V221F, V225I, and S226R; or the M protein includes any one or more site mutations of N32S, M33A, N49D, M51R, H54Y, A133T, V221F, V225I, and S226R; or the M protein includes any one or more site mutations of N32S, N49D, M51R, H54Y, A133T, V221F, V225I, and S226R.

Compared to an amino acid sequence shown in SEQ ID NO 12, the G protein includes any one or more site mutations of V53I, A141V, D172Y, K217E, D232G, V331A, V371E, G436D, T438S, F453L, T471I, and Y487H.

Compared to an amino acid sequence shown in SEQ ID NO 14, the N protein includes any one or more site mutations of I14V, R155K, and S353N.

Compared to an amino acid sequence shown in SEQ ID NO 16, the P protein includes any one or more site mutations of R50K, V76A, D99E, L126S, L140S, H151Y, I168M, K170E, Y189S, and N237D.

Compared to an amino acid sequence shown in SEQ ID NO 18, the L protein includes any one or more site mutations of S87P and I487T.

In the present application, an M protein in a wild-type Indiana MuddSummer subtype strain of VSV includes an amino acid sequence as shown in SEQ ID NO 1.

In a specific embodiment, the M protein includes an amino acid sequence as shown in SEQ ID NO 2.

In a specific embodiment, the M protein includes an amino acid sequence as shown in SEQ ID NO 3.

In a specific embodiment, the M protein includes an amino acid sequence as shown in SEQ ID NO 4.

In a specific embodiment, the M protein includes an amino acid sequence as shown in SEQ ID NO 5.

In a specific embodiment, the M protein includes an amino acid sequence as shown in SEQ ID NO 6.

In a specific embodiment, the M protein includes an amino acid sequence as shown in SEQ ID NO 7.

In a specific embodiment, the M protein includes an amino acid sequence as shown in SEQ ID NO 8.

In a specific embodiment, the M protein includes an amino acid sequence as shown in SEQ ID NO 9.

In a specific embodiment, the M protein includes an amino acid sequence as shown in SEQ ID NO 10.

In a specific embodiment, the M protein includes an amino acid sequence as shown in SEQ ID NO 11.

In the present application, a G protein in the wild-type Indiana MuddSummer subtype strain of the VSV includes an amino acid sequence as shown in SEQ ID NO 12.

In a specific embodiment, the G protein includes an amino acid sequence as shown in SEQ ID NO 13.

In the present application, an N protein in the wild-type Indiana MuddSummer subtype strain of the VSV includes an amino acid sequence as shown in SEQ ID NO 14.

In a specific embodiment, the N protein includes an amino acid sequence as shown in SEQ ID NO 15.

In the present application, a P protein in the wild-type Indiana MuddSummer subtype strain of the VSV includes an amino acid sequence as shown in SEQ ID NO 16.

In a specific embodiment, the P protein includes an amino acid sequence as shown in SEQ ID NO 17.

In the present application, an L protein in the wild-type Indiana MuddSummer subtype strain of the VSV includes an amino acid sequence as shown in SEQ ID NO 18.

In a specific embodiment, the L protein includes an amino acid sequence as shown in SEQ ID NO 19.

Preferably, the oncolytic virus is one or more selected from a group consisting of rhabdovirus, poxvirus, herpes simplex virus, measles virus, Semliki Forest virus, poliovirus, reovirus, Seneca Valley virus, Echo-type enterovirus, coxsackievirus, Newcastle disease virus, and Maraba virus.

In some specific embodiments, the recombinant oncolytic virus is obtained by carrying out site-directed mutagenesis on the rhabdovirus.

In some specific embodiments, the recombinant oncolytic virus is obtained by carrying out site-directed mutagenesis on the vesicular stomatitis virus (VSV).

In some specific embodiments, the recombinant oncolytic virus is obtained by carrying out site-directed mutagenesis on an Indiana MuddSummer subtype strain of the VSV.

Further, the recombinant oncolytic virus further includes an antigen encoded by an exogenous gene.

Further, the antigen is selected from a solid tumor antigen or a hematological tumor antigen.

Further, the solid tumor antigen includes but is not limited to 5T4, RORI, EGFR, FcγRI (CD64), FcγRIIa (CD32a), FcγRIIb (CD32b), CD28, CD137 (4-1BB), CTLA-4, HER2, HER3, FAS, FAP (fibroblast activation protein), LGR5, C5aR1, A2AR, fibroblast growth factor receptor 1 (FGFR1), FGFR2, FGFR3, FGFR4, glucocorticoid-induced TNFR-related (GITR) protein, lymphotoxin-β receptor (LTβR), tumor necrosis factor-related apoptosis-inducing ligand receptor 1 (TRAIL receptor 1), TRAIL receptor 2, prostate-specific membrane antigen (PSMA) protein, prostate stem cell antigen (PSCA) protein, tumor-related protein carbonic anhydrase IX (CAIX), epidermal growth factor receptor 1 (EGFR1), EGFRvIII, ErbB3 (HER3), folate receptor, ephrin receptor, PDGFRa, ErbB-2, CD2, CD40, CD74, CD80, CD86, CCAM5 (CD66e), CCAM6 (CD66c), p53, MET (tyrosine protein kinase Met), HGFR, MAGE-A1, MAGE-A2, MAGE-A3, MAGE-A4, MAGE-A6, MAGE-A10, MAGE-A12, BACE, DAM-6, DAM-10, GAGE-1, GAGE-2, GAGE-8, GAGE-3, GAGE-4, GAGE-5, GAGE-6, GAGE-7B, NA88-A, NY-ESO-1, BRCA1, BRCA2, MART-1, MC1R, Gp100, PSA, PSM, tyrosinase, TRP-1, TRP-2, ART-4, CAMEL, Cyp-B, hTERT, hTRT, iCE, MUC2, P-cadherin, myostatin (GDF8), Cripto (TDGF1), MUC5AC, PRAME, P15, RU1, RU2, SART-1, SART-3, AFP, β-catenin/m, caspase-8/m, CDK-4/m, ELF2M, GnT-V, G250, HSP70-2M, HST-2, KIAA0205, MUM-1, MUM-2, MUM-3, myosin/m, RAGE, SART-2, TRP-2/INT2, 707-AP, annexin II, CDC27/m, TPI/mbcr-abl, ETV6/AML, LDLR/FUT, Pml/RARα, TEL/AML1, CD28, CD137, CanAg, Mesothelin (MSLN), DR5, PD-1, PD-L1, IGF-1R, CXCR4, neuropilin 1 (NRP-1), glypican2/3 (GPC2/3), EphA2, B7-H3, B7-H4, gpA33, GPC3, SSTR2, GD2, VEGF-A, VEGFR-2, PDGFR-a, ANKL, RANKL, MSLN, EBV, TROP2, FOLR1, AXL, Claude 18.2, MUC1, TPBG, CEA, and EpCAM.

Further, the hematological tumor antigen includes but is not limited to BCMA (TNFRSF17), CD4, CD5 (Leu-1), CD7, CD10, FcγRIIIa (CD16a), FcγRIIIb (CD16b), CD19, CD20 (MS4A1), CD22 (Siglec-2), CD23, CD30 (TNFRSF8), CD33 (Siglec-3), CD34, CD37, CD38, CD44, CD47, CD56 (NCAM1), CD70, CD117, CD123 (IL3RA), CD138 (SDC1), CD174, CLL-1, ROR1, NKG2DL1/2 (ULBP1/2), IL1R3 (IL-1-RAP), FCRL5, GPRC5D, CLEC12A, WT1, FLT3, TLR8, SHP2, KAT6A/B, CSNK1A1, FLI1, IKZF1/3, PI3K, c-Kit, SLAMF3 (CD229), SLAMF7 (CD319), TCR B-chain, ITGB7, k-1gG, TACI, TRBCI, and LeY.

Further, the antigen is one or more selected from a group consisting of: CD19, CD22, BCMA, MUC1, NY-ESO-1, MAGE A4, MET, Claude 18.2, MSLN, EGFR, VEGFR2, HER2, TPBG, AFP, and MAGE-A10.

In a specific embodiment, the antigen CD19 includes an amino acid sequence as shown in SEQ ID NO 20.

In a specific embodiment, the antigen CD22 includes an amino acid sequence as shown in SEQ ID NO 21.

In a specific embodiment, the antigen NY-ESO-1 includes an amino acid sequence as shown in SEQ ID NO 22.

In a specific embodiment, the antigen MAGE A4 includes an amino acid sequence as shown in SEQ ID NO 23.

In a specific embodiment, the antigen Claude 18.2 includes an amino acid sequence as shown in SEQ ID NO 24.

In a specific embodiment, the antigen EGFR includes an amino acid sequence as shown in SEQ ID NO 25.

In a specific embodiment, the antigen HER2 includes an amino acid sequence as shown in SEQ ID NO 26.

Further, the small-molecule anticancer drug is any one or more of the following:
a small-molecule anticancer drug targeting ALK, including but not limited to Alectinib, Brigatinib, Ceritinib, Crizobtinib, Entrectinib, Lorlatinib, and Ensartinib;
a small-molecule anticancer drug targeting BTK, including but not limited to Acalabrutinib, Ibrutinib, Zanubrutinib, and Orelabrutinib;
a small-molecule anticancer drug targeting EGFR, including but not limited to Afatinib, Dacomitinib, Erlotinib, Gefitinib, Icotinib, Lapatinib, Mobocertinib, Osimertinib, Rybrevant, Befotertinib, Rezivertinib, Dasatinib, Brigatinib, Vandetanib, Almonetinib, and Furmonertinib;
a small-molecule anticancer drug targeting FGFR, including but not limited to Erdafitinib, Infigratinib, Pemazyre, Pemigatinib, Lenvatinib, Pazopanib, Anlotinib, and Ponatinib;
a small-molecule anticancer drug targeting HER2, including but not limited to Neratinib, Pyrotinib, Tucatinib, and Mobocertinib;
a small-molecule anticancer drug targeting Parp, including but not limited to Avapritinib, Fluzoparib, Niraparib, Olaparib, and Rucaparib;
a small-molecule anticancer drug targeting PI3K, including but not limited to duvelisib and linperlisib;
a small-molecule anticancer drug targeting VEGFR, including but not limited to Apatinib, Axitinib, Lenvatinib, Pazopanib, Regorafenib, Anlotinib, Cabozantinib, Sunitinib, Vandetanib, Ponatinib, Sorafenib, Donafenib, Surufatinib, and Fruquintinib;
a small-molecule anticancer drug targeting CDK4/6, including but not limited to Abemaciclib, Dalpiciclib, Palbociclib, and Ribociclib;
a small-molecule anticancer drug targeting KRAS, including but not limited to small-molecule anticancer drugs targeting KRAS G12A, KRAS G12C, KRAS G12D, KRAS G12R, KRAS G12V, KRAS G13D, KRAS Q61L and KRAS Q61H;
a small-molecule anticancer drug targeting KRAS G12C, including but not limited to Sotorasib and Adagrasib;
a small molecule anticancer drug indicated for melanoma, including but not limited to Vemurafenib, Dabrafenib, Encorafenib, Trametinib, Binimetmib, and Cobimetinib;
a small molecule anticancer drug indicated for cytoma or sarcoma, including but not limited to Pexidartinib, tazemetostat, Pazopanib, and Anlotinib;
a small molecule anticancer drug indicated for leukemia, including but not limited to Imatinib, Bosutinib, Nilotinib, Gilteritinib, and Ivosidenib; and
a small-molecule anticancer drug indicated for NSCLC, including but not limited to Sotorasib, Capmatinib, Tepotinib, Savolitinib, Pralsetinib, selpercatinib, Alectinib, Brigatinib, Ceritinib, Crizobtinib, Entrectinib, Lorlatinib, Afatinib, Dacomitinib, Erlotinib, Gefitinib, Icotinib, Mobocertinib, Osimertinib, Rybrevant, and Befotertinib.

Further, the small-molecule anticancer drug is one or more selected from a group consisting of: tivazanib, Everolimus, Sirolimus, Temsirolimus, Midostaurin, Ripretinib, Selumetinib, Larotrectinib, Lurbinectedin, and Olverembatinib.

Further, the small-molecule anticancer drug is any one or more of the following:
a single-target small-molecule anticancer drug, including but not limited to: Lorlatinib, Acalabrutinib, Ibrutinib, Zanubrutinib, Erlotinib, Gefitinib, Icotinib, Osimertinib, Rybrevant, Befotertinib, Rezivertinib, Erdafitinib, Infigratinib, Pemazyre, Pemigatinib, Pyrotinib, Tucatinib, Avapritinib, Fluzoparib, Niraparib, Olaparib, Rucaparib, linperlisib, Apatinib, Abemaciclib, Dalpiciclib, Palbociclib, Ribociclib, Vemurafenib, Dabrafenib, Encorafenib, Trametinib, Binimetmib, Cobimetinib, Pexidartinib, tazemetostat, Gilteritinib, Ivosidenib, Sotorasib, Capmatinib, Tepotinib, Savolitinib, Pralsetinib, selpercatinib, Everolimus, Sirolimus, Temsirolimus, Midostaurin, Ripretinib, Selumetinib, Larotrectinib, and Lurbinectedin; and
a multi-target small-molecule anticancer drug, including but not limited to: Alectinib, Brigatinib, Ceritinib, Crizobtinib, Entrectinib, Afatinib, Dacomitinib, Lapatinib, Mobocertinib, Dasatinib, Neratinib, duvelisib, Axitinib, Lenvatinib, Pazopanib, Regorafenib, Anlotinib, Cabozantinib, Sunitinib, Vandetanib, Ponatinib, Sorafenib, Imatinib, Bosutinib, Nilotinib, and tivazanib.

Further, the small-molecule anticancer drug is one or more selected from a group consisting of: Ceritinib, Ibrutinib, Afatinib, Dacomitinib, Icotinib, Lenvatinib, Pazopanib, Anlotinib, Pyrotinib, Niraparib, Olaparib, Regorafenib, Palbociclib, Vemurafenib, Savolitinib, Everolimus, Mobocertinib, Sotorasib and a combination thereof.

Further, the recombinant oncolytic virus also includes a cytokine encoded by an exogenous gene.

Further, the cytokine is selected from interleukins (IL), interferons (IFN), tumor necrosis factors (TNF), colony stimulating factors (CSF), transforming growth factor-β family (TGF-β family), and chemokine family.

Further, the cytokine is one or more selected from a group consisting of: GM-CSF, G-CSF, M-CSF, IL-1, IL-2, IL-4, IL-5, IL-6, IL-9, IL-10, IL-12, IL-13, IL-15, IL-17, IL-18, IL-23, IL-27, IFN-a, IFN-β, IFN-γ, IFN-β, TGF-β, and TNF-α.

Further, the cytokine is one or more selected from a group consisting of: GM-CSF, IL-2, IL-12, IL-15, IL-18, TNF-α, and IFN-β.

In a specific embodiment, the cytokine IL-12A includes an amino acid sequence as shown in SEQ ID NO 27.

In a specific embodiment, the cytokine IL-12B includes an amino acid sequence as shown in SEQ ID NO 28.

In a specific embodiment, the cytokine IL-18 includes an amino acid sequence as shown in SEQ ID NO 29.

In some specific embodiments, the recombinant oncolytic virus includes a nucleic acid molecule; the nucleic acid molecule includes a nucleic acid sequence encoding the M protein with the site mutation(s), a nucleic acid sequence encoding the G protein with the site mutation(s), a nucleic acid sequence encoding the N protein with the site mutation(s), a nucleic acid sequence encoding the P protein with the site mutation(s), and a nucleic acid sequence encoding the L protein with the site mutation(s).

In a specific embodiment, the ALK includes an amino acid sequence as shown in SEQ ID NO 30.

In a specific embodiment, the BTK includes an amino acid sequence as shown in SEQ ID NO 31.

In a specific embodiment, the FGFR1 includes an amino acid sequence as shown in SEQ ID NO 32.

In a specific embodiment, the VEGFR includes an amino acid sequence as shown in SEQ ID NO 33.

In a specific embodiment, the CDK4 includes an amino acid sequence as shown in SEQ ID NO 34.

In a specific embodiment, the CDK6 includes an amino acid sequence as shown in SEQ ID NO 35.

In a specific embodiment, the BRAF includes an amino acid sequence as shown in SEQ ID NO 36.

In a specific embodiment, the MET includes an amino acid sequence as shown in SEQ ID NO 37.

In a specific embodiment, the KRAS G12C includes an amino acid sequence as shown in SEQ ID NO 38.

In a specific embodiment, the nucleic acid molecule further includes a nucleic acid sequence encoding the cytokine.

In a specific embodiment, the nucleic acid molecule further includes a nucleic acid sequence encoding the antigen.

In a specific embodiment, the nucleic acid sequence encoding the cytokine is located between the nucleic acid sequence encoding the G protein with the site mutation(s) and the nucleic acid sequence encoding the L protein with the site mutation(s).

In a specific embodiment, the nucleic acid sequence encoding the antigen is located between the nucleic acid sequence encoding the G protein with the site mutation(s) and the nucleic acid sequence encoding the L protein with the site mutation(s).

An exogenous gene sequence of the antigen inserted into the recombinant oncolytic virus may be a complete sequence or a partial specific sequence. Similarly, a target sequence inserted into the recombinant oncolytic virus may be a complete sequence or a partial specific sequence. Similarly, an exogenous gene sequence of the cytokine inserted into recombinant oncolytic virus may be a complete sequence or a partial specific sequence.

In some specific embodiments, the method for treatment of a tumor by using a recombinant oncolytic virus in combination with a small-molecule anticancer drug is used for killing an abnormally proliferative cell in a sustained manner.

In some specific embodiments, the abnormally proliferative cell is selected from tumor cells or tumor tissue-related cells.

In some specific embodiments, the tumor includes solid tumors or hematological tumors.

In some specific embodiments, the tumor includes but is not limited to acute lymphoblastic leukemia, acute B-cell leukemia, chronic non-lymphocytic leukemia, non-Hodgkin's lymphoma, anal cancer, astrocytoma, basal cell carcinoma, bile duct cancer, bladder cancer, mastocarcinoma, breast cancer, cervical cancer, chronic myeloproliferative tumors, colorectal cancer, endometrial cancer, ependymoma, esophageal cancer, diffuse large B-cell lymphoma (DLBCL), sensorineuroblastoma, Ewing's sarcoma, fallopian tube cancer, gallbladder cancer, gastric cancer, gastrointestinal carcinoid tumors, hepatocellular carcinoma, hypopharyngeal cancer, Kaposi sarcoma, kidney cancer, Langerhans cell hyperplasia, laryngeal cancer, liver cancer, lung cancer, melanoma, Merkel cell carcinoma, mesothelioma, oral cancer, neuroblastoma, non-small cell lung cancer, osteosarcoma, ovarian cancer, pancreatic cancer, pancreatic neuroendocrine tumors, pharyngeal cancer, pituitary tumors, prostate cancer, rectal cancer, renal cell carcinoma, retinoblastoma, skin cancer, small cell lung cancer, small intestine cancer, squamous neck cancer, testicular cancer, thymoma, thyroid cancer, uterine cancer, vaginal cancer and vascular tumors.

The present application further provides a composition; the composition includes the oncolytic virus vaccine and the small-molecule anticancer drug.

In summary, the present application has the following beneficial effects:

The method for treatment of a tumor by using a recombinant oncolytic virus in combination with a small-molecule anticancer drug provided by the present application has obvious inhibitory effect on tumor growth. Moreover, the test results of using the recombinant oncolytic virus in direct combination with the small-molecule anticancer drug to treat tumors are superior to the test results of using the recombinant oncolytic virus or the small-molecule anticancer drug alone. The test results of using the recombinant oncolytic virus with insertion and expression of an antigen fragment in combination with the small-molecule anticancer drug to treat tumors are superior to the test results of using the recombinant oncolytic virus in direct combination with the small-molecule anticancer drug or the test results of directly using the recombinant oncolytic virus with insertion and expression of the antigen fragment. Therefore, the method for treatment of a tumor by using a recombinant oncolytic virus in combination with a small-molecule anticancer drug according to the present application further effectively improves the treatment effect on tumor cells.

The method for treatment of a tumor by using a recombinant oncolytic virus in combination with a small-molecule anticancer drug provided by the present application has good inhibitory effect on the growth of tumor cells. It can be learnt that the method for treatment of a tumor by using a recombinant oncolytic virus in combination with a small-molecule anticancer drug provided by the present application also has good inhibitory effect on other cancer cells, thus having broad clinical application prospects.

### BRIEF DESCRIPTION OF DRAWINGS

FIG. 1 shows the results of an animal experiment using a recombinant oncolytic virus in combination with small-molecule anticancer drug Mobocertinib to treat tumors.
FIG. 2 shows the results of an animal experiment using a recombinant oncolytic virus in combination with small-molecule anticancer drug Ceritinib to treat tumors.
FIG. 3 shows the results of an animal experiment using a recombinant oncolytic virus in combination with small-molecule anticancer drug Ibrutinib to treat tumors.
FIG. 4 shows the results of an animal experiment using a recombinant oncolytic virus in combination with small-molecule anticancer drug Afatinib to treat tumors.
FIG. 5 shows the results of an animal experiment using a recombinant oncolytic virus in combination with small-molecule anticancer drug Dacomitinib to treat tumors.
FIG. 6 shows the results of an animal experiment using a recombinant oncolytic virus in combination with small-molecule anticancer drug Icotinib to treat tumors.
FIG. 7 shows the results of an animal experiment using a recombinant oncolytic virus in combination with small-molecule anticancer drug Lenvatinib to treat tumors.
FIG. 8 shows the results of an animal experiment using a recombinant oncolytic virus in combination with small-molecule anticancer drug Pazopanib to treat tumors.
FIG. 9 shows the results of an animal experiment using a recombinant oncolytic virus in combination with small-molecule anticancer drug Anlotinib to treat tumors.
FIG. 10 shows the results of an animal experiment using a recombinant oncolytic virus in combination with small-molecule anticancer drug Pyrotinib to treat tumors.
FIG. 11 shows the results of an animal experiment using a recombinant oncolytic virus in combination with small-molecule anticancer drug Niraparib to treat tumors.
FIG. 12 shows the results of an animal experiment using a recombinant oncolytic virus in combination with small-molecule anticancer drug Olaparib to treat tumors.
FIG. 13 shows the results of an animal experiment using a recombinant oncolytic virus in combination with small-molecule anticancer drug Regorafenib to treat tumors.
FIG. 14 shows the results of an animal experiment using a recombinant oncolytic virus in combination with small-molecule anticancer drug Palbociclib to treat tumors.
FIG. 15 shows the results of an animal experiment using a recombinant oncolytic virus in combination with small-molecule anticancer drug Vemurafenib to treat tumors.
FIG. 16 shows the results of an animal experiment using a recombinant oncolytic virus in combination with small-molecule anticancer drug Savolitinib to treat tumors.
FIG. 17 shows the results of an animal experiment using a recombinant oncolytic virus in combination with small-molecule anticancer drug Sotorasib to treat tumors.

Those skilled in the art can easily perceive other aspects and advantages of the present application from the following detailed description. Only exemplary embodiments of the present application are shown and described in the following detailed description. As those skilled in the art will appreciate, the teachings of the present application enable those skilled in the art to make modifications to the specific embodiments disclosed without departing from the spirit and scope of the invention to which the present application relates. Accordingly, the drawings and descriptions in the specification of the present application are merely illustrative and not restrictive.

### DESCRIPTION OF EMBODIMENTS

The implementation of the present application will be described below by means of specific embodiments. Those skilled in the art can easily understand other advantages and effects of the present application from the contents described herein.

### DEFINITIONS

The term "oncolytic virus", as used herein, generally refers to a virus that can replicate in tumor cells and kill tumor cells. Oncolytic viruses include, but are not limited to, vesicular stomatitis virus (VSV), poxvirus, herpes simplex virus (HSV), measles virus, Semliki Forest virus, poliovirus, reovirus, Seneca Valley virus (SVV), Echo-type enterovirus, coxsackievirus, Newcastle disease virus (NDV), and Maraba virus. In certain embodiments, the oncolytic virus is modified to improve the selectivity for tumor cells. In certain embodiments, the oncolytic virus is modified to reduce its immunogenicity.

In some embodiments, the VSV is a mutant of the Indiana MuddSummer subtype strain of the VSV and can be used to treat a tumor. This virus does not interact with endogenous IFN-β in normal cells and can only selectively amplify and grow in tumor cells.

The VSV can express a variety of cell surface molecules, including low-density lipoprotein receptors, phosphatidylserine, sialolipids, and heparan sulfate, and can attach to the cell surface by these molecules. Compared with other oncolytic virus platforms currently under development, the VSV has the following advantages: (1) small genome, short replication time, and fast transsynaptic speed; (2) extremely high expression of exogenous genes, resulting in high titers and a possibility of large-scale production; and (3) independent cell cycle and no risk of transformation in the cytoplasm of host cells. This oncolytic virus will not integrate into DNA, and after being attenuated, this oncolytic virus can avoid the nervous system inflammation caused by wild-type viruses. In view of the above characteristics, the VSV has great potential in tumor immunotherapy.

In some embodiments, the M protein, and/or the G protein, and/or the N protein, and/or the P protein, and/or the L protein of the VSV may be subjected to site-directed mutagenesis.

In certain embodiments, the recombinant oncolytic virus described herein may be an oncolytic virus that has been genetically modified, such as modified by modification of one or more genes, so as to improve its tumor selectivity and/or preferentially replicate in dividing cells. The "genetically modified" may refer to modification of genes involved in DNA/RNA replication, nucleic acid metabolism, host tropism, surface attachment, virulence, lysis and diffusion, or modification of integrating exogenous genes. The exogenous genes may include exogenous immune regulatory genes, exogenous screening genes and exogenous reporter genes. The oncolytic virus that has been modified may also be an oncolytic virus modified at an amino acid level, such as, by insertion, deletion, or substitution of one or more amino acids.

The term "M protein", as used herein, generally refers to a matrix protein of the VSV. The M protein is an important virulence factor of the VSV and is also a protein in the VSV that is known to interfere with the natural immune response of mice. The term "M protein" also encompasses homologs, orthologs, variants and functionally active fragments thereof. In the present application, the M protein in a wild-type Indiana MuddSummer subtype strain of the VSV may include an amino acid sequence as shown in SEQ ID NO 1. In the present application, the M protein of the oncolytic virus may include amino acid sequences as shown in SEQ ID NOs 2-11.

The term "G protein", as used herein, generally refers to a glycoprotein of the VSV, also known as the envelope protein. The term "G protein" also encompasses homologs, orthologs, variants and functionally active fragments thereof. In the present application, the G protein in a wild-type Indiana MuddSummer subtype strain of the VSV may include an amino acid sequence as shown in SEQ ID NO 12. In the present application, the G protein of the oncolytic virus may include an amino acid sequence as shown in SEQ ID NO 13.

The term "N protein", as used herein, generally refers to a nucleocapsid protein of the VSV. The term "N protein" also encompasses homologs, orthologs, variants and functionally active fragments thereof. In the present application, the N protein in Indiana MuddSummer subtype of a wild-type VSV may include an amino acid sequence as shown in SEQ ID NO 14. In the present application, the N protein of the oncolytic virus may include an amino acid sequence as shown in SEQ ID NO 15.

The term "P protein", as used herein, generally refers to a phosphoprotein of the VSV. The term "P protein" also encompasses homologs, orthologs, variants and functionally active fragments thereof. In the present application, the P protein in a wild-type Indiana MuddSummer subtype strain of the VSV may include an amino acid sequence as shown in SEQ ID NO 16. In the present application, the P protein of the oncolytic virus may include an amino acid sequence as shown in SEQ ID NO 17.

The term "L protein", as used herein, generally refers to an RNA poly E protein of the VSV. An L gene of the VSV encodes the RNA poly E protein. The term "L protein" also encompasses homologs, orthologs, variants and functionally active fragments thereof. In the present application, the L protein in a wild-type Indiana MuddSummer subtype strain of the VSV may include an amino acid sequence as shown in SEQ ID NO 18. In the present application, the L protein of the oncolytic virus may include an amino acid sequence as shown in SEQ ID NO 19.

In the present application, the mutated site of a protein is generally expressed as "amino acid + amino acid position + amino acid after mutation". In the present application, the mutation may include but is not limited to the addition, substitution absence and/or deletion of an amino acid. For example, the term "M51R" generally refers to the mutation of methionine (M) at position 51 to arginine (R).

In the present application, the term "amino acid substitution" generally refers to the substitution of an amino acid residue present in a parent sequence with another amino acid residue. The amino acid in the parent sequence may be substituted, for example, via chemical synthesis or by recombinant methods known in the art. Therefore, "substitution at position xx" generally refers to the substitution of an amino acid present at position xx with an alternative amino acid residue. In the present application, the amino acid substitution may include an amino acid mutation.

In the present application, the term "mutation" generally refers to a change in the nucleotide or amino acid sequence of a wild-type molecule. Amino acid changes may include the substitution, deletion, absence, insertion, addition or truncation of an amino acid, or the processing or cleavage of a protein.

In the present application, the recombinant oncolytic virus means integration of an exogenous gene at the same time of site-directed mutagenesis on the M protein, and/or G protein, and/or N protein, and/or P protein, and/or L protein of the VSV. The exogenous gene specifically refers to a gene encoding an antigen and/or a cytokine.

In the present application, the term "small-molecule anticancer drug" refers to chemical substances or polypeptides, and typically refers to signaling inhibitors, which are called small-molecule anticancer drugs because of their small molecular weight. Small-molecule anti-cancer drugs are usually made into oral preparations. After being taken orally, these small-molecule anti-cancer drugs can quickly dissolve in the intestines. After being absorbed by the human body, they bind to the active conformation of certain kinases to inhibit the activity of the kinases and prevent the growth of cancer cells; or they block the passage of signals mediated in tumor cells and inhibit the activity of abnormal proteins to prevent the growth and spread of cancer cells.

ALK is a receptor tyrosine kinase. Activated ALK can activate downstream ALK/PLCV, JAK/STAT, MEK/ERK and PI3K/AKT pathways, thereby promoting cell cycle progression, proliferation and angiogenesis, which in turn leads to tumor occurrence. Small-molecule anticancer drugs targeting ALK can inhibit ALK phosphorylation and ALK-mediated activation of downstream signaling proteins STAT3 and AKT, suppress ALK fusion and amplification, and reduce tumor cell viability.

BTK is a tyrosine kinase that exists in B cells and is the core center for B-cell growth signaling. Studies have found that BTK is a key protein for the rapid increase of malignant B cells. Small-molecule anticancer drugs targeting BTK can inactivate BTK, inhibit B-cell growth signaling, and can relatively accurately inhibit the growth of malignant B cells, so that the number of malignant B cells can be controlled.

EGFR is a glycoprotein and is a member of receptor tyrosine kinase family. It penetrates the cell membrane. After binding to a ligand and being activated, the transmembrane receptor on the cell converts from monomer to dimer, activating downstream pathways within the cell and inducing cell proliferation. Studies have shown that EGFR is highly or abnormally expressed in many solid tumors. Activating mutations in EGFR lead to structural activation of the tyrosine kinase, phosphorylation of downstream pathways, and ultimately lead to uncontrolled cell proliferation. Small-molecule anticancer drugs targeting EGFR can inhibit the activity of EGFR, thereby blocking the activation of related pathways.

Alterations in FGFR molecules can lead to abnormal FGF/FGFR signaling, promote cell proliferation, neovascularization, invasion, metastasis, anti-apoptosis, etc., which is associated with a wide range of human malignancies. Common mutations in FGFR include gene amplification/fusion/deletion mutations. The FGFR family mainly includes four isoforms: FGFR1, FGFR2, FGFR3 and FGFR4, as well as some isomeric molecules. Each isoform has the structural characteristics of an extracellular region that binds to ligands, a transmembrane region, and an intracellular region where the receptor phosphorylation occurs and is a part of the tyrosine kinase signaling pathway responsible for cell proliferation and differentiation. They form ternary complexes with 18 different fibroblast growth factors (FGFs), which in turn initiate a series of signaling pathways and participate in regulating physiological processes in organisms, such as embryonic and fetal development, wound healing and angiogenesis. Small-molecule anticancer drugs targeting FGFR can inhibit the activity of FGFR, thereby blocking related signaling pathways.

The transmembrane receptor protein tyrosine kinase encoded by HER2 consists of three parts: an extracellular ligand-binding domain, a single-chain transmembrane domain and an intracellular protein tyrosine kinase activation domain. The extracellular domain forms a homodimer (HER2-HER2) or heterodimer (HER2-EGFR), which mediates the phosphorylation of the intracellular tyrosine kinase activation domain, thereby in turn activating the downstream signaling pathways and promoting cell proliferation and angiogenesis. The HER2 protein of the HER2-encoding gene product is usually only expressed during the fetus and is only expressed at low levels in a very few tissues after adulthood. The HER2 on the surface of HER2-positive cells is 10-100 times that of normal cells. Studies have shown that amplification/overexpression of the HER2 gene exists in more than 30% of human tumors, such as breast cancer, ovarian cancer, endometrial cancer, fallopian tube cancer, gastric cancer and prostate cancer. The small-molecule anticancer drugs targeting HER2 are oral non-peptide anilinoquinazolone compounds homologous to ATP. This similarity allows them to compete for the ATP-binding domain of protein kinases, thereby preventing phosphorylation and subsequent activation of signaling pathways and further leading to apoptosis and reduced cell proliferation.

Parp is a DNA repair enzyme that plays a key role in the single-stranded DNA repair pathway. The specific process is as follows: Parp is activated when DNA is damaged and broken, with the function of recognizing and binding to the site of DNA breaks, Parp then activates and catalyzes the polyADP ribosylation of receptor proteins and participates in the DNA repair process. Small-molecule anticancer drugs targeting Parp can block the above-mentioned repair process, leaving damaged DNA unrepaired and causing cells to die. When single-strand repair is blocked in normal cells, another repair pathway, namely homologous recombination repair, is triggered. Therefore, small-molecule anticancer drugs targeting Parp are not very toxic to normal cells, but for some tumor cells, If the homologous recombination repair function also fails, small-molecule anticancer drugs targeting Parp can selectively kill tumor cells with BRCA1/2 gene deletion, which is called "synthetic death".

The PI3K/Akt/mTOR signaling pathway is involved in regulating the proliferation, survival, transcription, translation and metabolism of malignant tumor cells. Small-molecule anticancer drugs targeting PI3K can act on lymphoma cells, inhibit PI3K expression, and reduce AKT protein phosphorylation level, thereby inducing apoptosis of malignant B lymphocytes and inhibiting proliferation of malignant B lymphocytes.

VEGFR is a receptor for VEGF and is expressed in lymph and vascular endothelium. VEGFR1/2/3 regulates the proliferation and migration of endothelial cells, is highly expressed in many tumors, and plays an important role in tumor angiogenesis. Inhibition of VEGFR has become an effective treatment for many cancers. Small-molecule anticancer drugs targeting VEGFR can block the VEGF signaling pathway by binding to VEGF or interfering with different VEGFR domains, thereby inhibiting the proliferation and migration of endothelial cells.

CDK4/6 is a group of protein kinases discovered for its regulatory role in the cell cycle and is also believed to be involved in transcriptional regulation, mRNA processing, and neural cell differentiation. CDK4 and CDK6 in cyclin-dependent kinases can form complexes with cyclin D, thereby phosphorylating RB protein (retinoblastoma protein), and in turn promoting cell proliferation by releasing the transcription factor E2F1. Small-molecule anticancer drugs targeting CDK4/6 can effectively reduce the phosphorylation of Rb-protein, down-regulate the expression of E2F, cause cell cycle arrest, and inhibit cell proliferation.

More than 90% of chronic myeloid leukemia cases are caused by a chromosomal abnormality called the Philadelphia chromosome. The Philadelphia chromosome refers to a translocation involving chromosomes 9, 22. This chromosome translocation will cause the abl proto-oncogene originally located on chromosome 9 to be transferred to the bcr gene on chromosome 22, forming a bcr-abl fusion gene. The protein originally expressed by the abl gene is a tyrosine kinase located in the cell. The translocated bcr gene will affect the regulation of the abl gene. The bcr-abl fusion protein binds to GRB2 and SRC proteins to activate the Ras-Raf pathway, which leads to growth factor-independent cell amplification, activates the PI3K pathway to inhibit programmed cell death, activates the CRKL-FAK-PYK2 pathway to reduce cell surface viscosity, thereby enhancing the ability of cancer cells to metastasize. And the bcr-abl fusion protein participates in signaling and transcription of activated JAK-STAT pathway, etc. Eventually, it leads to continuous proliferation and metastasis of cancer cells with the abnormal bcr-abl gene, which in turn presents leukemia. In addition to inhibiting abl kinase, small-molecule anticancer drugs indicated for leukemia can also inhibit Kit and related signaling pathways such as growth factor PDGFR.

JAK is a non-receptor tyrosine protein kinase with four isoforms: JAK1, JAK2, JAK3 and TYK2. The three isoforms of JAK1, JAK2 and TYK2 are widely found in various tissues and cells in the body. JAK3 is only distributed in the bone marrow and lymphatic system. The JAK-STAT signaling pathway is indispensable for both immune and hematopoietic functions. Various different pro-inflammatory factors activate this intracellular signaling pathway to cause inflammatory reactions, and therefore it has now become a new target in the fields of immune inflammation, cancer and other fields. Small-molecule anticancer drugs targeting JAK-STAT signaling pathway was developed for inhibiting the JAK-STAT signaling pathway.

Thanks to the GTP hydrolysis activity, KRAS protein can function as a binary switch downstream of cell surface receptors during signal transduction. Simply put, the nucleotide binding state of KRAS determines its activation state. In the absence of mitotic signaling, the KRAS protein mainly binds to GDP and is in an inactive conformation. This inactive state is maintained by intrinsic GTP hydrolysis activity and interaction with GTP enzyme activating protein (GAP), thereby accelerating the conversion of GTP to GDP. When activated by mitotic signaling, cell-surface receptors recruit guanine nucleotide exchange factors (GEF) to bind to inactive KRAS and catalyze the expulsion of GDP from the active site, thereby passively loading GTP. The binding of GTP to KRAS changes the active site from an open conformation to a closed conformation, and the closed conformation promotes subsequent interactions between KRAS and various effector proteins. KRAS is one of the most common oncogenes in solid tumors. About 30% of tumors have KRAS mutations, including 90% of pancreatic cancers, 30% to 40% of colon cancers, and 15% to 20% of lung cancers. Among the KRAS gene mutations, 97% occur at amino acid residue 12 or 13, including G12C, G12D, G13D, and the G12C mutation is the most common. KRAS G12C mutation occurs in approximately 14% of lung adenocarcinomas (the most common subtype of NSCLC), 4% of colorectal cancers, and 2% of pancreatic cancers. Patients with this mutation have a poor prognosis and are prone to resistance to standard therapies. Once chemotherapy or immunotherapy fails, treatment options are very limited. As the "most difficult target", currently approved inhibitors for the KRAS G12C mutation include Lumakras (sotorasib) and Krazati (adagrasib).

Adagrasib is a specific inhibitor against the KRAS G12C mutation and has shown good efficacy in the treatment of non-small cell lung cancer, colorectal cancer and other solid tumors.

In some specific embodiments, the small-molecule anticancer drugs may include but be not limited to:
1. Small-molecule anticancer drugs targeting ALK (Anaplastic Lymphoma Kinase):
   Alectinib (Roche) targets ALK and RET, the indications of which may be ALK-positive NSCLC (non-small cell lung cancer).
   Brigatinib (Ariad Pharmaceuticals) targeting ALK, ROS1, IGF-1R, FLT3, and EGFR, the indications of which may be ALK-positive NSCLC.
   Ceritinib (Novartis) targeting ALK, IGF-1R, InsR and ROS1, the indications of which may be ALK-positive NSCLC.
   Crizobtinib (Pfizer) targeting ALK, c-Met, HGFR, ROS1, and MST1R, the indications of which may be ALK- and ROS1-positive NSCLC.
   Entrectinib (Roche) targeting TRKA/B/C, ROS1 and ALK, the indications of which may be ROS1-positive NSCLC and solid tumors with fusion expression of NTRK protein.
   Lorlatinib (Pfizer) targeting ALK, the indications of which may be ALK-positive NSCLC.
   Ensartinib targeting ALK.
2. Small-molecule anticancer drugs targeting BTK (Bruton Tyrosine Kinase)
   Acalabrutinib (AstraZeneca) targeting BTK, the indications of which may be mantle cell lymphoma, CLL (chronic lymphocytic leukemia), and SLL (small lymphocytic lymphoma).
   Ibrutinib (Pharmacyclics) BTK, the indications of which may be mantle cell lymphoma, CLL and Waldenstrom's macroglobulinemia.
   Zanubrutinib (BeOne Medicines) targeting BTK, the indications of which may be mantle cell lymphoma.
   Orelabrutinib targeting BTK.
3. Small-molecule anticancer drug targeting EGFR (Epidermal Growth Factor Receptor)
   Afatinib (Boehringer Ingelheim) targeting EGFR, ERBB2 and ERBB4, the indications of which may be NSCLC2013 and squamous NSCLC2016.
   Dacomitinib (Pfizer) targeting EGFR, ERBB and ERBB4, the indications of which may be EGFR-mutant NSCLC.
   Erlotinib (OSI Pharmaceuticals) targeting EGFR, the indications of which may be NSCLC and pancreatic cancer.
   Gefitinib (AstraZeneca) targeting EGFR, the indications of which may be NSCLC.
   Icotinib (Betta Pharmaceuticals) targeting EGFR, the indications of which may be NSCLC.
   Lapatinib (Smithkline Beecham) targeting EGFR and ErbB2, the indications of which may bebreast cancer.
   Mobocertinib (Takeda Pharmaceuticals, Inc.) targeting EGFR and HER2, the indications of which may be locally advanced or metastatic NSCLC with EGFR exon 20 insertions.
   Osimertinib (AstraZeneca) targeting EGFR T970M, the indications of which may be NSCLC with EGFR-sensitive mutations.
   Rybrevant (Janssen) targeting EGFR, the indications of which may be NSCLC with EGFR exon 20 insertions.
   Befotertinib (Betta Pharmaceuticals) targeting EGFR, the indications of which may be EGFR TKI-resistant T790M-mutant NSCLC.
   Rezivertinib (Beta Pharma) targeting EGFR, the indications of which may be NSCLC.
   Dasatinib (Bristol-Myers Squibb) targeting bcr-abl, EGFR, Src, Lck, Yes, Fyn, Kit, EphA2 and PDGFRβ, the indications of which may be chronic myelogenous leukemia.
   Almonetinib (Hansoh Pharma) targeting EGFR.
   Furmonertinib (Allist Pharmaceuticals) targeting EGFR.
4. Small-molecule anticancer drugs targeting FGFR (Fibroblast Growth Factor Receptor)
   Erdafitinib (Janssen Biotech) targeting FGFR1/2/3/4, the indications of which may be urothelial bladder cancer.
   Infigratinib (QED Therapeutics, Inc.) targeting FGFR2, the indications of which may be advanced or metastatic FGFR2 fused cholangiocarcinoma.
   Pemazyre (Incyte) targeting FGFR1/2/3, the indications of which may be advanced cholangiocarcinoma.
   Pemigatinib (Incyte) targeting FGFR1/2/3, the indications of which may be local advanced or metastatic cholangiocarcinoma with fusion or rearrangement of FGFR2.
5. Small-molecule anticancer drugs targeting HER2 (Human Epidermal Growth Factor Receptor)
   Neratinib (Puma Pharmaceuticals) targeting ERBB2 and HER2, the indications of which may be HER2-positive breast cancer.
   Pyrotinib (Hengrui Pharmaceuticals) targeting HER2, the indications of which may be solid tumors.
   Tucatinib (Seattle Genetics) targeting HER2, the indications of which may be unresectable or metastatic advanced HER2-positive breast cancer.
6. Small-molecule anticancer drugs targeting Parp (Poly(adenosine diphosphate-ribose) polymerase)
   Avapritinib (Blueprint Medicines Corp) targeting Parp, the indications of which may be gastrointestinal stromal tumors with mutations in exon 18 of the PDGFR gene.
   Fluzoparib (Hengrui Pharmaceuticals) targeting Parp, the indications of which may be platinum-sensitive recurrent ovarian cancer, fallopian tube cancer or primary peritoneal cancer, platinum-sensitive recurrent epithelial ovarian cancer, fallopian tube cancer, with germline BRCA mutations after previous second-line or above chemotherapy.
   Niraparib (ZEJULA Tesaro, Inc.) targeting Parp, the indications of which may be maintenance treatment of epithelial ovarian cancer, fallopian tube cancer and primary peritoneal cancer that respond fully/partially to first-line platinum chemotherapy.
   Olaparib (AstraZeneca) targeting Parp, the indications of which may be HRD-positive advanced ovarian cancer, fallopian tube cancer and primary peritoneal cancer.
   Rucaparib (Rubraca clovis onology, Inc.) targeting Parp, the indications of which may be prostate cancer after androgen receptor and taxane chemotherapy.
7. Small-molecule anticancer drugs targeting PI3K (phosphatidyl inositol-3-hydroxykinase)
   Duvelisib (CSPC) targeting dual inhibitors of PI3K-δ and PI3K-γ, the indications of which may be relapsed/refractory follicular lymphoma.
   Linperlisib (YL-Pharma), a highly selective inhibitor targeting PI3K-δ, the indications of which may be relapsed/refractory follicular lymphoma.
8. Small-molecule anticancer drugs targeting VEGFR (Vascular Endothelial Growth Factor Receptor)
   Apatinib (Hengrui Pharmaceuticals) targeting VEGFR2, the indications of which may be gastric cancer 2014 and liver cancer 2020.
   Axitinib (Pfizer) targeting VEGFR1/2/3, PDGFRβ, the indications of which may be RCC (renal cell carcinoma).
   Lenvatinib (Alche Pharmaceuticals) targeting VEGFR, FGFR, PDGFR, KIT and RET, the indications of which may be DTC (differentiated thyroid cancer).
   Pazopanib (GlaxoSmithKline) targeting VEGFR1/2/3, PDGFRα/β, FGFR1/3, Kit, LCk, Fms and Itk, the indications of which may be renal cancer and soft tissue sarcoma.
   Regorafenib (Bayer Group) targeting VEGFR1/2/3, bcr-abl, B-Raf, B-RafyV600E and Kit, the indications of which may be CRC (colorectal cancer) and GIST (gastrointestinal stromal tumor).
   Anlotinib (Chiatai Tianqing), a multi-target tyrosine kinase inhibitor capable of effectively acting on VEGFR, PDGFR, FGFR, c-Kit and other targeting and having dual effects of anti-tumor angiogenesis and inhibiting tumor growth, the indications of which may be NSCLC, soft tissue sarcoma, and small cell lung cancer. In addition, an indication of Chiatai Tianqing for the treatment of medullary thyroid tumors may soon be approved.
   Cabozantinib (Exelixis Biopharmaceutical) targeting RET, Met, VEGFR1/2/3, Kit, TrkB, Flt3, Axl, Tie2 and ROS1, the indications of which may be metastatic medullary thyroid carcinoma, advanced kidney cancer and liver cancer.
   Sunitinib (Pfizer) targeting PDGFRa, VEGFR1/2/3, Kit, Flt3, CSF-1R and RET, the indications of which may be RCC, GIST, PNET (primitive neuroectodermal tumors).
   Vandetanib (IPR PHARMS Inc.) targeting EGFRs, VEGFRs, RET, Brk, Tie2, EphRs and Srcfamily kinases, the indications of which may be medullary thyroid cancer.
   Ponatinib (ARIAD Pharmaceuticals) targeting bcr-abl, bcr-abl T315I, VEGFR, PDGFR, FGFR, EphR, Src, Kit, RET, Tie2 and Flt3, the indications of which may be chronic granulocytic leukemia.
   Sorafenib (Bayer Pharmaceuticals) targeting B/C-Raf, B-RafV600E, Kit, Flt3, RET, VEGFR 1/2/3 and PDGFR β, the indications of which may be hepatocellular carcinoma, RCC and DTC.
   Donafenib targeting VEGFR and PDGFR.
   Surufatinib targeting VEGFR 1/2/3, FGFR, and CSF-IR.
   Fruquintinib targeting VEGFR 1/2/3.
9. Small-molecule anticancer drugs targeting CDK4/6 (Cyclin Dependent Kinase 4/6)
   Abemaciclib (Lilly) targeting CDK4/6, the indications of which may be HR+, HER-breast cancer.
   Dalpiciclib (Hengrui Pharmaceuticals) targeting CDK4/6, which may be used in combination with fulvestrant for hormone receptor-positive (HR+), HER2 recurrent or metastatic breast cancer that has progressed after previous endocrine therapy.
   Palbociclib (Pfizer) targeting CDK4/6, the indications of which may be ER+, HER- breast cancer.
   Ribociclib (Novartis Pharmaceuticals) targeting CDK4/6, the indications of which may be HR+, EGFR- breast cancer.
10. Small-molecule anticancer drugs indicated for melanoma
   Vemurafenib (Hoffman) targeting BRAF, the indications of which may be B-RafV600E mutant melanoma.
   Dabrafenib (GlaxoSmithKline) targeting B-Raf, the indications of which may be melanoma, NSCLC and BRAF-mutant anaplastic thyroid cancer.
   Encorafenib (Array Biopharmaceutical) targeting B-Raf-V600E/K, the indications of which may be B-Raf-V600E/K mutant melanoma.
   Trametinib (GlaxoSmithKline) targeting MEK1/2, the indications of which may be melanoma and BRAF-mutant NSCLC.
   Binimetmib (Array Biopharmaceutical) targeting MEK1/2, the indications of which may be B-RafV600E/K mutant melanoma.
   Cobimetinib (Genentech) targeting MEK1/2, the indications of which may be BRAF-V600E/K mutant melanoma.
11. Small-molecule anticancer drugs indicated for cytoma or sarcoma
   Pexidartinib (Daiichi Sankyo) targeting CSFR1/Kit, and the indication can be tenoshex giant cytoma.
   Tazemetostat (Epizyme) targeting EZH2 mutations, the indications of which may be epithelioid sarcoma/follicular lymphoma.
12. Small-molecule anticancer drugs indicated for leukemia
   Imatinib (Novartis Pharmaceutical) targeting A/B/C-Raf, B-RafV600E, SRMS, ACK1, MAP4K5 and FGR, the indications of which may be chronic myeloid leukemia, aggressive systemicmastosis, CEL (chronic eosinophilic leukemia), GIST, and MDS (myelodysplastic syndrome).
   Bosutinib (Wyeth) targeting bcr-abl, Src, Lyn and Hck, the indications of which may be chronic myeloid leukemia.
   Nilotinib (Novartis Pharmaceuticals) targeting ber-abl/PDGFR and DDR1, the indications of which may be chronic myeloid leukemia.
   Gilteritinib (Astellas) targeting FLT3, the indications of which may be FLT3-mutated AML (acute myeloid leukemia).
   Ivosidenib (Cstone Pharmaceuticals) targeting mutant isocitrate dehydrogenase, the indications of which may be recurrent or refractory IDH1 acute myeloid leukemia.
13. Small-molecule anticancer drugs indicated for NSCLC (non-small cell lung cancer)
   Sotorasib (Amgen) targeting KRAS G12C, the indications of which may be KRAS G12C mutant NSCLC.
   Capmatinib (Novartis) targeting MET, the indications of which may be metastatic NSCLC carrying MET14 exon skipping.
   Tepotinib (Merck) targeting MET, the indications of which may be metastatic NSCLC and gastric and esophageal cancer with MET exon 14 skipping.
   Savolitinib (Hutchison Whampoa) targeting MET, the indications of which may be advanced NSCLC with MET exon 14 skipping.
   Pralsetinib (Blueprint/Cstone) targeting RET, the indications of which may be RET fusion-positive metastatic non-NSCLC.
   Selpercatinib (Loxo Oncology, Inc.)) targeting RET, the indications of which may be RET fusion-positive NSCLC and medullary thyroid carcinoma.
14. Other small-molecule anticancer drugs
   Tivazanib (AVEO), a multi-enzyme inhibitor, the indications of which may be RCC.
   Everolimus (Novartis Pharmaceuticals) targeting FKBP12/mTOR, the indications of which may be HER2- breast cancer, PNET, RCC, and RAML (renal angiomyolipoma).
   Sirolimus (Wyeth) targeting FKBP12/mTOR, the indications of which may be renal transplant lymphangioleiomyomatosis.
   Temsirolimus (Wyeth) targeting FKBP12/mTOR, the indications of which may be advanced kidney cancer.
   Midostaurin (Novartis Pharmaceuticals) targeting FLT3, the indications of which may be FLT3-mutated AML.
   Ripretinib (Deciphera) targeting KIT/PDGFRα, the indications of which may be GIST.
   Selumetinib (Smetinib, AstraZeneca) targeting MEK1/2, the indications of which may be symptomatic, inoperable plexiform neurofibromatosis type 1.
   Larotrectinib (Lilly) targeting NTRK, the indications of which may be solid tumors with NTRK protein fusion.
   Lurbinectedin (GlaxoSmithKline) targeting alkylating agents, the indications of which may be adult metastatic small cell lung cancer that progresses during or after platinum-based chemotherapy.
   Olverembatinib (Ascentage), a small-molecule protein tyrosine kinase inhibitor, capable of effectively inhibiting the activity of wild-type and multiple mutant forms of Bcr-Abl tyrosine kinase, inhibiting the phosphorylation of Bcr-Abl tyrosine kinase and downstream proteins STAT5 and Crkl, blocking downstream pathway activation, and inducing cell cycle arrest and apoptosis in Bcr-Abl positive, Bcr-Abl T315I-mutant cell lines, the indications of which may be Bcr-Abl, KIT leukemia, and gastrointestinal stromal tumor.
15. Small-molecule anticancer drugs targeting KRAS
   Sotorasib targeting KRAS G12C.
   Adagrasib targeting KRAS G12C.

In this application, the term "antigen" refers to a substance that can cause the production of antibodies or immune cells, and is any substance that can induce an immune response in the body. That is, the antigen is a substance that can be specifically recognized and bound by the antigen receptor (TCR/BCR) on the surface of T/B lymphocytes, activate T/B cells, cause them to proliferate and differentiate, produce immune response products (sensitized lymphocytes or antibodies), and can specifically bind to the corresponding products in vivo and in vitro. Therefore, antigenic substances have two important characteristics: immunogenicity and immunoreactivity. Immunogenicity refers to the ability of antigens to induce specific immune responses in the body and produce antibodies and/or sensitized lymphocytes. Immunoreactivity refers to the ability to specifically bind to corresponding immune effector substances (antibodies or sensitized lymphocytes) in vivo and in vitro.

In some specific embodiments, the antigen is exogenous, meaning that the antigen comes from a different species.

In some specific embodiments, the antigen is an endogenous antigen. Specifically, the antigen is an antigen that is usually expressed on tumor cells.

In a specific embodiment, the antigen is a tumor associated antigen (TAA) or a tumor specific antigen (TSA).

In a specific embodiment, the TAA or TSA encompasses molecules or portions thereof that are presented on the cell surface (antigens recognized by CARs) or within the cell membrane (antigens recognized by TCRs) or present in the tumor environment (e.g., in the tumor microenvironment).

In some specific embodiments, the cell is a tumor cell.

In some specific embodiments, the TAA or TSA includes a tumor associated antigen or a tumor specific antigen on the cell surface or in the cell membrane.

In some specific embodiments, the cell is a non-tumor cell present in a tumor environment. The cell is, for example, but not limited to, a cell present in the vasculature tissue associated with a tumor or cancer.

In some specific embodiments, the TAA or TSA is an angiogenic antigen in a tumor microenvironment.

In some specific embodiments, the TAA or TSA is an antigen on a blood vessel in a tumor microenvironment.

In some specific embodiments, the cell is a stromal cell present in a tumor environment.

In some specific embodiments, the TAA or TSA is a stromal cell antigen in a tumor microenvironment.

In some specific embodiments, the TAA or TSA includes an extracellular epitope of a tumor cell surface antigen, a tetramer inside or outside the tumor cell membrane, or other structures that can be recognized by antibodies or immune cells.

In some specific embodiments, the TAA or TSA includes an extracellular matrix antigen.

In some specific embodiments, the TAA or TSA includes an antigen present in a tumor microenvironment (TME).

In some specific embodiments, the TAA or TSA includes a molecule secreted by a tumor cell into a TME.

In some specific embodiments, the TAA or TSA includes an effector molecule secreted by a tumor cell into a TME.

In some specific embodiments, the TAA or TSA includes an effector molecule secreted by a tumor cell into a TME to downregulate or inhibit the activity of cytotoxic natural killer (NK) cells or T cells.

In some specific embodiments, the TAA or TSA includes a soluble activating receptor ligand that is secreted by a tumor cell into a TME to block recognition of tumor cells by NK cells or T cells.

In some specific embodiments, examples of TAA or TSA include but are not limited to 5T4, ROR1, EGFR, FcγRI, FcγRIIa, FcγRIIb, FcγRIIIa, FcγRIIIb, CD28, CD137, CTLA-4, FAS, FAP (fibroblast activation protein), LGR5, C5aR1, A2AR, fibroblast growth factor receptor 1 (FGFR1), FGFR2, FGFR3, FGFR4, glucocorticoid-induced TNFR-related (GITR) protein, lymphotoxin-β receptor (LTβR), toll-like receptor (TLR), tumor necrosis factor-related apoptosis-inducing ligand receptor 1 (TRAIL receptor 1), TRAIL receptor 2, prostate-specific membrane antigen (PSMA) protein, prostate stem cell antigen (PSCA) protein, tumor-related protein carbonic anhydrase IX (CAIX), epidermal growth factor receptor 1 (EGFR1), EGFRvIII, human epidermal growth factor receptor 2 (HER2/neu; Erb2), ErbB3 (Her3), folate receptor, ephrin receptor, PDGFRa, ErbB2, CD2, CD20, CD22, CD30, CD33, CD40, CD37, CD38, CD70, CD74, CD56, CD80, CD86, CD123, CCAM5, CCAM6, BCMA, p53, MET (tyrosine protein kinase Met), hepatocyte growth factor receptor (HGFR), MAGE-A1, MAGE-A2, MAGE-A3, MAGE-A4, MAGE-A6, MAGE-A10, MAGE-A12, BAGE, DAM-6, DAM-10, GAGE-1, GAGE-2, GAGE-8, GAGE-3, GAGE-4, GAGE-5, GAGE-6, GAGE-7B, NA88-A, NY-ESO-1, BRCA1, BRCA2, MART-1, MCIR, Gp100, PSA, PSM, tyrosinase, Wilms tumor antigen (WT1), TRP-1, TRP-2, ART-4, CAMEL, Cyp-B, hTERT, hTRT, iCE, MUC1, MUC2, P-cadherin, myostatin (GDF8), Cripto (TDGF1), MUC5AC, PRAME, P15, RU1, RU2, SART-1, SART-3, WT1, AFP, β-catenin/m, caspase-8/m, CDK-4/m, ELF2M, GnT-V, G250, HSP70-2M, HST-2, KIAA0205, MUM-1, MUM-2, MUM-3, myosin/m, RAGE, SART-2, TRP-2/INT2, 707-AP, annexin II, CDC27/m, TPI/mbcr-abl, ETV6/AML, LDLR/FUT, Pml/RARa, TEL/AML1, CD28, CD137, CanAg, mesothelin, DR5, PD-1, PD-L1, HER2, HER3, IGF-1R, CXCR4, neuropilin 1, glypicans, EphA2, CD138, B7-H3, B7-H4, gpA33, GPC3, SSTR2 or VEGF-R2, CEA, and EpCAM.

The term "cytokines", as used herein, refers to biologically active substances synthesized and secreted by immune cells (such as lymphocytes, monocytes and macrophages) and their related cells (such as vascular endothelial cells and fibroblasts) to regulate the functions of other immune cells or target cells. The cytokines belong to small molecule polypeptides or glycoproteins. Cytokines with immune regulation effects can be expressed by recombinant oncolytic viruses. According to their main functions, cytokines are divided into: interleukins (IL), interferons (IFN), tumor necrosis factors (TNF), colony stimulating factors (CSF), transforming growth factor-β family (TGF-β family), growth factors (GF), and chemokine family.

Interleukins include IL-1, IL-2, IL-7, IL-9, IL-15, IL-21, IL-4, IL-12 and IL-18.

Specifically, interleukin 1 (IL-1): IL-1 is a pleiotropic cytokine involved in cortical inflammatory response, cell growth and tissue repair. The IL-1 superfamily has 11 members, such as IL-1A, IL-1B, IL-1Ra, and IL-18. IL-1 is a drug target for some cancers and is also used in cell therapy. In terms of cellular immunotherapy, IL-1 can stimulate the proliferation of CD4+ T cells in vitro, induce the production of IL-2, co-stimulate the activation of CD8+/IL1R+ T cells, and stimulate the proliferation of mature B cell and the secretion of immunoglobulin.

Specifically, interleukin 2 (IL-2): IL-2, also known as T cell growth factor, is produced by T cells in response to antigens or mitotic stimulation and is widely used to promote the activation and proliferation of T cells and NK cells. IL-2 can stimulate proliferation of NK cells, increase cytotoxicity, and stimulate NK cells to secrete a variety of cytokines. However, further studies have found that IL-2 can cause overdifferentiation of T cells and induce apoptosis of activated T cells, and can also activate CD4+FoxP3Treg regulatory cells, thereby inhibiting activation of T cells and tumor killing activity. Therefore, IL-2 is considered to be a T cell regulatory factor rather than just an activation factor, so some studies have now used IL-7, IL-15, and IL-21 to replace IL-2.

Specifically, interleukin 7 (IL-7): IL-7 is a hematopoietic growth factor, secreted by stromal cells in the bone marrow and thymus, and shares the γc receptor subunit with IL-2 to stimulate the proliferation of lymphocyte progenitor cells. IL-7 can provide continuous stimulation signals for Naive T cells and memory T cells. As mentioned above, IL-7 will not activate CD4+ FoxP3+ Treg cells during the activation of CD8+ T cells. In clinical practice, IL-7 can also be used to restore the number of T cells after chemotherapy or hematopoietic stem cell transplantation. And IL-7 plays an important role in certain stages of maturation and can affect the proliferation of B cells. IL-7 can also act as a regulatory factor for intestinal mucosal lymphocytes.

Specifically, interleukin 15 (IL-15): IL-15 has a similar structure to IL-2, shares the γc receptor subunit, and belongs to the family with 4 α-helix bundles (others include, such as, IL-2, IL-4, IL-7, IL-9, G-CSF and GM-CSF). IL-15 regulates the activation and proliferation of T cells and NK cells. IL-15 mainly kills virus-infected cells in the innate immune system. Also, IL-15 can activate NKT cells and γδT cells. In immune cell therapy, IL-15 does not cause apoptosis of activated T cells, but activates CD8+ effector T cells. IL-15 maintains the survival of memory T cells, thus playing an important role in long-term anti-tumor activity.

Specifically, interleukin 21 (IL-21): IL-21 also belongs to the IL-2 family, shares the γc receptor subunit, has a strong regulatory effect on the cells of the immune system, and can induce cell division and proliferation in its target cells. In cellular immunotherapy, IL-21 can promote the proliferation of CD4+ and CD8+T cells, enhance the cytotoxicity of CD8+T cells and NK cells, and will not cause cell apoptosis due to activation. IL-21 will preferentially expand "young" CD8+ T cells with CD27+ and CD28+, which have stronger cytotoxicity. Of course, IL-21 will not cause the expansion of Treg, so IL-21 is increasingly used in cell immunotherapy.

Specifically, interleukin 4 (IL-4): IL-4 activates the proliferation of activated B cells and T cells, and regulates the expression of Fc receptors on lymphocytes and monocytes. IL-4 induces the transformation of Th1 cells to Th2 cells. IL-4 stimulates Th2 cells to secrete IL-4, IL-5, IL-6, IL-10 and IL-13. IL-4 guides monocytes to differentiate into DCs by inhibiting the growth of macrophages. When IL-4 is not added to a culture system, monocytes will differentiate into macrophages. IL-4 plays a key role in regulating humoral immunity and adaptive immunity, inducing B cell antibody class switching to IgE and upregulating the production of MHC class II molecules. The combined action of IL-4 and GM-CSF can direct the differentiation of monocytes into immature DCs. At this time, DCs have stronger antigen uptake and processing capabilities, but weaker antigen presentation capabilities. The sequential use of IL-4 and TNF-α can promote DC maturation.

Specifically, interleukin 12 (IL-12): IL-12 acts on activated T and NK cells, has a wide range of biological activities, and acts on lymphocytes through the mediation of the activator of the transcription protein STAT4. IL-12 is required for the T cell-independent induction of IFN-γ and plays an important role in the differentiation of Th1 and Th2 cells. IL12B combines with IL23A to form IL-23 interleukin, which has innate and adaptive immune functions. IL-12 is a drug target. In cellular immunotherapy, IL-12 promotes the differentiation of CD4+ T cells into CD4+ Th1 T cells and enhances the activity of CD8+ CTLs cells. The curative effect of IL-12 is related to its dose, duration of action, and other interacting cytokines, and promotes the tumor-killing activity of immune cells through multiple mechanisms. In the mouse anti-melanoma model, high-dose IL-12 acts through NK cells, while low-dose IL-12 has a tumor-killing effect through NKT.

Specifically, interleukin 18 (IL-18): IL-18 is also called interferon-γ inducing factor, which is a proinflammatory cytokine produced by macrophages and other cells. IL-18 can stimulate NK cells and CD8+ T cells to secrete IFN-γ, and enhance the cytotoxic effect of NK cells and CD8+ T cells. IL-18 can also activate macrophages, promote the development of Th1 CD4+ T cells and promote the expression of FasL by lymphocytes. IL-18 can provide a potential therapeutic target for allergic diseases. In addition, the synergistic effect of IL-18, IL-12 and IL-15 can maintain Th1 response and monokine production in autoimmune diseases.

Interferon-γ (IFN-γ) belongs to type II interferon, which is mainly produced by NK and NKT cells, has antiviral, antitumor and immunomodulatory effects and includes IFN-γ and IFN-β. IFN-γ has an anti-proliferative effect on transformed cells and can enhance the antiviral and anti-tumor effects of type I interferon. By activating macrophages, IFN-γ induces the expression of MHC I, MHCIII and co-activator molecules on antigen presenting cells (APCs). In addition, IFN-γ can induce changes in the expression of proteasomes to enhance antigen presentation ability. IFN-γ can also promote the differentiation of CD4+ T cells into Th1 cells and inhibit the subtype switching of B cells that depends on IL-4. IFN-γ activates the JAK-STAT cell pathway by phosphorylating JAK1 and JAK2 proteins. In cellular immunotherapy, IFN-γ acts on host immune cells and has certain effects on macrophages, T cells, B cells and NK cells. IFN-γ enhances antigen presentation ability by promoting the expression of MHC class II molecules in macrophages or by causing some cells that normally do not express MHC class II molecules (such as vascular endothelial cells, some epithelial cells and connective tissue cells) to express MHC class II molecules. IFN-γ can promote the differentiation of B cells and CD8+T cells, but cannot promote their proliferation. IFN-γ can enhance the activity and immune function of TH1 cells. IFN-γ enhances the phagocytic ability of neutrophils and activates NK cells to enhance their cytotoxic effect. Abnormal expression of IFN-γ is associated with many autoinflammatory and autoimmune diseases.

Tumor necrosis factor belongs to the TNF superfamily cytokines and is a multifunctional molecule that regulates biological processes, including cell proliferation, differentiation, apoptosis, lipid metabolism and coagulation. TNF-α participates in anti-tumor. In terms of cellular immunotherapy, TNF-α causes immature DC to differentiate into mature DC. This process is achieved by TNF-α downregulating the macropinocytosis of immature DC and the expression of surface Fc receptors, and upregulating the expression of MHC class I and class II molecules and B7 family molecules (CD80, CD86, etc.) on cell surface. The antigen uptake and processing capabilities of mature DCs are significantly weakened, but their antigen presentation capabilities are significantly enhanced, which can strongly activate T cells. TNF-α can also affect the production of other cytokines, such as stimulating the secretion of IL-1 by monocytes and macrophages, enhancing the proliferation of IL-2-dependent thymocytes and T cells, promoting the production of lymphokines such as IL-2, CSF and IFN-γ, and enhancing the proliferation and Ig secretion of B cells stimulated by mitogens or foreign antigens.

Granulocyte macrophage colony-stimulating factor (GM-CSF) plays a key role in embryo transfer and development. GM-CSF is one of the earliest cytokines discovered to have an effect on DCs. In DC culture, GM-CSF promotes the differentiation of monocytes into large macrophage-like cells and promotes the expression of MHC class II molecules on the cell surface, thereby enhancing the antigen presentation function of cells. In addition, GM-CSF can also promote DC survival. In terms of cellular immunotherapy, GM-CSF can activate immune responses and produce anti-tumor activity by activating macrophages and DCs. In terms of antigen presentation, GM-CSF can promote DC cell maturation, promote the upregulation of costimulatory molecules and the expression of CD1d receptors. Recent studies have found that GM-CSF stimulates the differentiation of hematopoietic progenitor cells into monocytes and neutrophils, thereby reducing the risk of febrile neutropenia in cancer patients. Other studies have shown that GM-CSF can induce the differentiation of bone marrow DCs, promote Th1 cell-biased immune responses and angiogenesis, and affect the development of allergic inflammation and autoimmune diseases. Therefore, GM-CSF is used clinically to treat malignant tumors.

In the present application, the term "nucleic acid molecule" generally refers to nucleotides of any length. In the present application, the term "nucleic acid molecule" may encode a protein included by the oncolytic virus. In the present application, the nucleic acid molecule may include DNA and/or RNA. In some cases, the RNA may include single-stranded RNA (ssRNA) or double-stranded RNA (dsRNA). The single-stranded RNA may include sense RNA or anti-sense RNA, or it may include ambisense RNA.

In the present application, the term "prevention" generally refers to preventing the occurrence and onset, recurrence, and/or spread of a disease or one or more symptoms of the disease by taking certain measures in advance. The term "treatment", as used herein, generally refers to eliminating or ameliorating a disease, or one or more symptoms associated with the disease. In certain embodiments, treatment generally refers to administering one or more drugs to a patient suffering from the disease so that the disease is eliminated or alleviated. In certain embodiments, "treatment" may be the administration of the pharmaceutical composition and/or drug product in the presence or absence of other drugs after the onset of symptoms of a particular disease. For example, it may be the use of the pharmaceutical composition and/or drug product described herein to prevent the occurrence, development, recurrence and/or metastasis of a tumor.

In the present application, the term "tumor" generally refers to any new pathological growth of tissue. The tumor may be benign or malignant. In the present application, the tumor may be a solid tumor or a hematological tumor. For research use, these tissues can be isolated from readily available sources by methods well known to those skilled in the art.

In some specific embodiments, the tumors include but are not limited to acute lymphoblastic leukemia, acute B-cell leukemia, chronic non-lymphocytic leukemia, non-Hodgkin's lymphoma, anal cancer, astrocytoma, basal cell carcinoma, bile duct cancer, bladder cancer, mastocarcinoma, breast cancer (BRCA), cervical cancer, chronic myeloproliferative tumors, colorectal cancer, endometrial cancer, ependymoma, esophageal cancer, diffuse large B-cell lymphoma (DLBCL), sensorineuroblastoma, Ewing's sarcoma, fallopian tube cancer, gallbladder cancer, gastric cancer, gastrointestinal carcinoid tumors, hepatocellular carcinoma, hypopharyngeal cancer, Kaposi sarcoma, kidney cancer, Langerhans cell hyperplasia, laryngeal cancer, liver cancer, lung cancer, melanoma, Merkel cell carcinoma, mesothelioma, oral cancer, neuroblastoma, non-small cell lung cancer, osteosarcoma, ovarian cancer, pancreatic cancer, pancreatic neuroendocrine tumors, pharyngeal cancer, pituitary tumors, prostate cancer, rectal cancer, renal cell carcinoma, retinoblastoma, skin cancer, small cell lung cancer, small intestine cancer, squamous neck cancer, testicular cancer, thymoma, thyroid cancer, uterine cancer, vaginal cancer and vascular tumors.

### DETAILED DESCRIPTION

A wild-type VSV, in particular, an Indiana strain of the VSV, Indiana MuddSummer subtype strain of the VSV, is provided. The Indiana MuddSummer subtype strain of the VSV includes: an M protein with an amino acid sequence as shown in SEQ ID NO 1; a G protein with an amino acid sequence as shown in SEQ ID NO 12; an N protein with an amino acid sequence as shown in SEQ ID NO 14; a P protein with an amino acid sequence as shown in SEQ ID NO 16; an L protein with an amino acid sequence as shown in SEQ ID NO 18. In the present application, the M protein, G protein, N protein, P protein, and L protein all can be modified.

A recombinant oncolytic virus is provided. The oncolytic virus is derived from the wild-type VSV described above and obtained by mutating the sites on the amino acid sequences of the M protein, G protein, N protein, P protein, and L protein of the wild-type VSV.

A method for treatment of a tumor by using a recombinant oncolytic virus in combination with a small-molecule anticancer drug is provided in the present application, which specifically includes: treating the tumor using the combination of the small-molecule anticancer drug and the recombinant oncolytic virus.

The small-molecule anticancer drug includes a small-molecule anticancer drug targeting ALK, a small-molecule anticancer drug targeting BTK, a small-molecule anticancer drug targeting EGFR, a small-molecule anticancer drug targeting FGFR, a small-molecule anticancer drug targeting HER2, a small-molecule anticancer drug targeting Parp, a small-molecule anticancer drug targeting PI3K, a small-molecule anticancer drug targeting VEGFR, a small-molecule anticancer drug targeting CDK4/6, and a small-molecule anticancer drug targeting KRAS.

The recombinant oncolytic virus includes an M protein, a G protein, an N protein, a P protein, and an L protein.

Compared to the amino acid sequence shown in SEQ ID NO 1, the M protein includes any one or more site mutations of M51R, V221F, and S226R; or the M protein includes any one or more site mutations of N32S, N49D, M51R, H54Y, V221F, V225I, and S226R; or the M protein includes any one or more site mutations of N32S, N49D, M51R, H54Y, knockouting of bases encoding leucine at position 111, V221F, V225I, and S226R; or the M protein includes any one or more site mutations of N32S, N49D, M51R, H54Y, L111A, V221F, V225I, and S226R; or the M protein includes any one or more site mutations of G21E, N32S, N49D, M51R, H54Y, V221F, V225I, and S226R; or the M protein includes any one or more site mutations of G21E, N32S, M33A, N49D, M51R, H54Y, V221F, V225I, and S226R; or the M protein includes any one or more site mutations of G21E, N32S, M33A, N49D, M51R, H54Y, A133T, V221F, V225I, and S226R; or the M protein includes any one or more site mutations of N32S, M33A, N49D, M51R, H54Y, V221F, V225I, and S226R; or the M protein includes any one or more site mutations of N32S, M33A, N49D, M51R, H54Y, A133T, V221F, V225I, and S226R; or the M protein includes any one or more site mutations of N32S, N49D, M51R, H54Y, A133T, V221F, V225I, and S226R.

Compared to the amino acid sequence shown in SEQ ID NO 12, the G protein includes any one or more site mutations of V53I, A141V, D172Y, K217E, D232G, V331A, V371E, G436D, T438S, F453L, T471I, and Y487H.

Compared to the amino acid sequence shown in SEQ ID NO 14, the N protein includes any one or more site mutations of I14V, R155K, and S353N.

Compared to the amino acid sequence shown in SEQ ID NO 16, the P protein includes any one or more site mutations of R50K, V76A, D99E, L126S, L140S, H151Y, I168M, K170E, Y189S, and N237D.

Compared to the amino acid sequence shown in SEQ ID NO 18, the L protein includes any one or more site mutations of S87P and I487T.

Further, the recombinant oncolytic virus is obtained by introducing an exogenous gene encoding the antigen into the recombinant oncolytic virus described above.

Further, the small-molecule anticancer drug is one or more selected from a group consisting of: Ceritinib, Ibrutinib, Afatinib, Dacomitinib, Icotinib, Lenvatinib, Pazopanib, Anlotinib, Pyrotinib, Niraparib, Olaparib, Regorafenib, Palbociclib, Vemurafenib, Savolitinib, Everolimus, Mobocertinib, and Sotorasib.

Further, the recombinant oncolytic virus further includes the antigen encoded by the exogenous gene.

Further, the antigen is one or more selected from a group consisting of: CD19, CD22, BCMA, MUC1, NY-ESO-1, MAGE A4, MET, Claude 18.2, MSLN, EGFR, VEGFR2, HER2, TPBG, AFP, and MAGE-A10.

Further, the recombinant oncolytic virus further includes a cytokine encoded by an exogenous gene.

Further, the cytokine is one or more selected from a group consisting of: GM-CSF, IL-2, IL-12, IL-15, IL-18, TNF-α, and IFN-β.

In the present application, the recombinant oncolytic virus described herein can be obtained through a virus packaging process and a virus rescue process. The specific process may include infecting and inoculating BSR-T7 cells with poxvirus vTF7-3 expressing T7 RNA polymerase, and performing lipofectamine transfection using expression plasmids that clone VSV N, VSV P, and VSV L genes, respectively, and backbone plasmids to obtain the oncolytic virus of interest.

The present application provides a composition; the composition includes the recombinant oncolytic virus and small-molecule anticancer drug described above.

In certain embodiments, the composition may include a suitable formulation of one or more (pharmaceutically effective) adjuvants, stabilizers, excipients, diluents, solubilizers, surfactants, emulsifiers and/or preservatives. The acceptable components of the composition are preferably non-toxic to a recipient at the dose and concentration used. The compositions of the present application include but are not limited to liquid, frozen and lyophilized compositions.

In certain embodiments, the pharmaceutically acceptable carrier may include any and all solvents, dispersion media, coatings, isotonic agents and absorption delaying agents that are compatible with drug administration, and are generally safe and non-toxic.

In certain embodiments, the composition may be useful for administration including parenteral, subcutaneous, intracavitary, intraarterial, intravenous, intrathecal, and/or intranasal administration or may be directly injected into tissues. For example, the composition may be administered to a patient or subject by infusion or injection. In certain embodiments, the administration of the composition may be performed in different ways, such as intravenous, intraperitoneal, subcutaneous, intramuscular, intradermal or intratissue administration. In certain embodiments, the composition may be administered uninterruptedly. The uninterrupted (or continuous) administration may be achieved by a small pump system worn by a patient to measure a therapeutic agent flowing into the patient's body, as described in WO2015/036583.

The present application further provides a use of the composition described above in preparing a medicine for preventing and/or treating a disease and/or disorder.

The method for treatment of a tumor by using a recombinant oncolytic virus in combination with a small-molecule anticancer drug provided by the present application has obvious inhibitory effect on tumor growth. Moreover, the test results of using the recombinant oncolytic virus in direct combination with the small-molecule anticancer drug to treat tumors are superior to the test results of using the recombinant oncolytic virus or the small-molecule anticancer drug alone. The test results of using the recombinant oncolytic virus with insertion and expression of an antigen fragment in combination with the small-molecule anticancer drug to treat tumors are superior to the test results of using the recombinant oncolytic virus in direct combination with the small-molecule anticancer drug or the test results of directly using the recombinant oncolytic virus with insertion and expression of the antigen fragment. Therefore, the method for treatment of a tumor by using a recombinant oncolytic virus in combination with a small-molecule anticancer drug provided by the present application further effectively improves the treatment effect on tumor cells.

The method for treatment of a tumor by using a recombinant oncolytic virus in combination with a small-molecule anticancer drug provided by the present application has good inhibitory effect on the growth of tumor cells. It can be learnt that the method for treatment of a tumor by using a recombinant oncolytic virus in combination with a small-molecule anticancer drug provided by the present application also has good inhibitory effect on other cancer cells, thus having broad clinical application prospects.

The present application is further described in detail below in combination with Preparation Examples 1-116 and Examples.

### PREPARATION EXAMPLES

### Preparation Examples 1-10

Preparation Examples 1-10 provide a method for treatment of a tumor by using a recombinant oncolytic virus in combination with a small-molecule anticancer drug, respectively.

In the method, the small-molecule anticancer drug and the recombinant oncolytic virus are used in combination to treat the tumor.

The recombinant oncolytic virus includes an M protein, a G protein, an N protein, a P protein and an L protein. The M protein, the G protein, the N protein, the P protein, and the L protein were all obtained by carrying out site-directed mutagenesis on the wild-type VSV virus Indiana MuddSummer subtype.

The preparation examples differ in the mutated sites of the M protein. Mutated sites of the M protein were amino acid sequences as shown in SEQ ID NO 2 to SEQ ID NO 11, respectively. The G protein included an amino acid sequence as shown in SEQ ID NO 13; the N protein included an amino acid sequence as shown in SEQ ID NO 15; the P protein included an amino acid sequence as shown in SEQ ID NO 17; and the L protein included an amino acid sequence as shown in SEQ ID NO 19.

The mutated sites of the proteins and the type of the small-molecule anticancer drug are shown in Table 1.

A construction method for the recombinant oncolytic virus provided by said preparation examples was as follows:

### (1) Construction of vector

Using a pRV-core plasmid (BioVector NTCC (National Type Culture Collection)) as a template, the mutated sites of the M protein, the mutated sites of the G protein, the mutated sites of the N protein, the mutated sites of the P protein and the mutated sites of the L protein shown in Table 1 were introduced by PCR.

Gene fragments containing XbaI and MluI restriction sites and including the protein mutated sites described above was synthesized respectively. PCR amplification was performed using the gene fragments as templates. The PCR products were subjected to 1% agarose gel electrophoresis and double-digested with XbaI and MluI, and a gene fragment with the mutated sites of the M protein, a gene fragment with the mutated sites of the G protein, a gene fragment with the mutated sites of the N protein, a gene fragments with the mutated sites of the P protein, a gene fragment with the mutated sites of the L protein were obtained by gel extraction using a gel extraction kit. The pRV-core plasmid was double-digested with XbaI and Mlu I, and a pRV-core restriction digestion extraction skeleton fragment was obtained by gel extraction using a gel extraction kit.

The gene fragment with the mutated sites of the M protein, the gene fragment with the mutated sites of the G protein, the gene fragment with the mutated sites of the N protein, the gene fragments with the mutated sites of the P protein and the gene fragment with the mutated sites of the L protein were respectively ligated to the pRV-core restriction digestion extraction skeleton fragment for transformation, the transformation product was then plated, and monoclones were selected and shaken for identification by PCR. The constructed plasmid pRV-core Mut was then obtained and was sequenced by a sequencing company.

**Table 1 Recombinant oncolytic viruses and small-molecule anticancer drugs in Preparation Examples 1-10**

| Prepa ration Exam ple | Recombinant oncolytic virus | | | | | | | | | | | | | | | Small -mole cule antica ncer drug |
|---|---|---|---|---|---|---|---|---|---|---|---|---|---|---|---|---|
| | M protein | | | G protein | | | N protein | | | P protein | | | L protein | | | |
| | Mutated sites | Numb er of mutati ons | Seq uenc e | Mut ated sites | Nu mbe r of mut atio | Se qu en ce | Mut ated sites | Nu mb er of mut | Se qu en ce | Mut ated site s | Nu mb er of mut | S eq ue nc e | Mut ated sites | Nu mb er of mu | S e q u e | Type |
| | | | | | ns | | | atio ns | | | atio ns | | | tati ons | n c e | |
| 1 | M51R, V221F, S226R | 3 | SEQ ID NO 2 | V53 I, A14 1V, D17 2Y, K21 7E, D23 2G, V33 1A, V37 1E, G43 6D, T43 8S, F45 3L, T47 1I, Y48 7H | 12 | S E Q ID N O 13 | 114V , R15 5K, S35 3N | 3 | S E Q ID N O 15 | R50 K, V76 A, D99 E, L12 6S, L14 0S, H15 1Y, I16 8M, K17 0E, Y18 9S, N23 7D | 10 | S E Q I D N O 1 7 | S87 P, I487 T | 2 | S E Q I D N O 1 9 | Mobo certin ib |
| 2 | N32S, N49D, M51R, H54Y, V221F, 2251, S226R | 7 | SEQ ID NO 3 | | | | | | | | | | | | | |
| 3 | N32S, N49D, M51R, H54Y, knockout of bases encoding leucine at position 111, V221F, V2251, S226R | 8 | SEQ ID NO 4 | | | | | | | | | | | | | |
| 4 | N32S, N49D, M51R, H54Y, L111A, V221F, V225I, S226R | 8 | SEQ ID NO 5 | | | | | | | | | | | | | |
| 5 | G21E, N32S, N49D, M51R, H54Y, V221F, V225I, S226R | 8 | SEQ ID NO 6 | | | | | | | | | | | | | |
| 6 | G21E, N32S, M33A, N49D, M51R, H54Y, V221F, V225I, S226R | 9 | SEQ ID NO 7 | | | | | | | | | | | | | |
| 7 | G21E, N32S, M33A, N49D, M51R, H54Y, A133T, V221F, V225I, S226R | 10 | SEQ ID NO 8 | | | | | | | | | | | | | |
| 8 | N32S, M33A, N49D, M51R, H54Y, 221F, V225I, S226R | 8 | SEQ ID NO 9 | | | | | | | | | | | | | |
| 9 | N32S, M33A, N49D, M51R, H54Y, 133T, V221F, 2251, S226R | 9 | SEQ ID NO 10 | | | | | | | | | | | | | |
| 10 | N32S, N49D, M51R, H54Y, A133T, 221F, V225I, S226R | 8 | SEQ ID NO 11 | | | | | | | | | | | | | |

### (2) Virus rescue

The constructed plasmid pRV-core Mut was transfected into a BSR-T7 cell (purchased from ATCC, American Type Culture Collection) by cell transfection technology using a calcium phosphate transfection kit (Thermo Fisher Scientific).

Four plasmids, i.e., the pRV-core Mut, pP, pN, and pL, were mixed at a ratio of 10:5:4:1, and the total amount of the plasmids was 5 µg; the plasmid mixture was then diluted with 200 µl of an Opti-MEM (Thermo Fisher Scientific), and 7.5 µl of a transfection reagent Plus Reagent (Life Technologies) was added to obtain a transfection plasmid premix, where the pP was a plasmid carrying the phosphoprotein genes of a rhabdovirus, the pN was a plasmid carrying the nucleoprotein genes of a rhabdovirus, and the pL was a plasmid carrying the polymerase protein genes of a rhabdovirus; parent vectors corresponding to the three plasmids pN, pP, and pL were all pCAGGS (purchased from ATCC).

10 µl of Lipofectamine LTX (Thermo Fisher Scientific) was diluted with 200 µl of the Opti-MEM to obtain an LTX mixed solution.

Plasmid transfection was performed according to the method described in the instruction manual of Lipofectamine LTX. Six hours later, the BSR-T7 cell was washed twice with PBS and then seeded in DMEM (Thermo Fisher Scientific) supplemented with 10% fetal bovine serum and incubated for three days.

The cell supernatant of the BSR-T7 cell culture solution was transferred to a Vero cell (Thermo Fisher Scientific), and the Vero cell was incubated at 37 °C for three days. The cells were observed for green fluorescence under a fluorescence microscope to determine the result of virus rescue. The rescued mutant rhabdovirus library was further passaged via the Vero cell, and monoclonal virus strains were selected in an established plaque screening system.

(3) Gene sequencing the viral genomic RNA was extracted using a Trizol kit, and reverse transcription was performed using random primers. The reverse transcribed cDNA was subjected to PCR using primers designed for the gene sequence of the M protein, primers designed for the gene sequence of the G protein, primers designed for the gene sequence of the N protein, primers designed for the gene sequence of the P protein, primers designed for the gene sequence of the L protein and primers designed for the gene sequence encoding the antigen.

The primer sequences designed for the gene sequence of the M protein were:
□ PF: ATGAGTTCCTTAAAGAA;
□ PR: TCATTTGAAGTGG.

The primer sequences designed for the gene sequence of the G protein were:
□ PF: ATGAAGTGCCTTTTGTACTTAG;
□ PR: TTACTTTCCAAGTCGGTTCATCT.

The primer sequences designed for the gene sequence of the N protein were:
□ PF: ATGTCTGTTACAGTCAAGAG;
□ PR: TCATTTGTCAAATTCTGACTT.

The primer sequences designed for the gene sequence of the P protein were:
□ PF: ATGGATAATCTCACAAAAGTTCG;
□ PR: CTACAGAGAATATTTGACTCTCG.

The primer sequences designed for the gene sequence of the L protein were:
□ PF: ATGGAAGTCCACGATTTTGAGA;
□ PR: TTAATCTCTCCAAGAGTTTTCCT.

The product was extracted after 1% agarose gel electrophoresis and sequenced by a sequencing company. The sequencing results are shown in Table 1.

### Preparation Examples 11-49

Preparation Examples 11-49 provide a method for treatment of a tumor by using a recombinant oncolytic virus in combination with a small-molecule anticancer drug, respectively.

In the method, the small-molecule anticancer drug and the recombinant oncolytic virus are used in combination to treat the tumor.

The recombinant oncolytic virus includes an M protein, a G protein, an N protein, a P protein, an L protein and an antigen. The mutated sites of the M protein, the G protein, the N protein, the P protein and the L protein are the same as the corresponding mutated sites in Preparation Example 2, and

the difference lies in the type of the antigen and the type of the small-molecule anticancer drug. The mutated sites of the proteins, the type of the antigen and the type of the small-molecule anticancer drug are shown in Table 2.

The construction method of the recombinant oncolytic viruses provided in said preparation examples is the same as the construction method in Preparation Example 2, except that:

Between the step (1) of construction of vector and the step (2) of virus rescue, the method further included the following step: insertion of an exogenous gene encoding the antigen. Specifically, the plasmid pRV-core Mut obtained in the step (1) was treated by double restriction digestion with Xho I and Mlu I to extract a long fragment. The exogenous gene encoding the antigen was synthesized by a gene synthesis company and amplified with corresponding primers. The amplification product was double-digested with Xho I and Nhe I and a target gene fragment (the double-digested exogenous gene fragment) was extracted. The double-digested pRV-core Mut and the double-digested exogenous gene fragment were ligated and transformed. Monoclones were selected and identified by PCR or enzyme digestion, and then sequenced by a sequencing company to obtain a plasmid pRV-core Mut carrying the exogenous gene. In the step (2) of vrus rescue, the plasmid pRV-coreMut carrying the exogenous gene was used to transfect BSR-T7 cells.

Step (3) included sequencing of each type of antigen. The viral genomic RNA was extracted using a Trizol kit, and reverse transcription was performed using random primers. The reverse transcribed cDNA was subjected to PCR using primers designed for gene sequence encoding the antigen.

The primer sequences designed for the gene sequence encoding the antigen were:
1) CD19 F: ACGCTCGAGATGCCACCTCCTCGCCTCC;
   CD19 R: TCTGGCTAGCTCATCTTTTCCTCCTCAGG.
2) CD22 F: ATGCATCTCCTCGGCCCCT;
   CD22 R: TCAGAGCCCACAGATTGCCAGG.
3) NY-ESO-1 F: ACGCTCGAGATGCAGGCAGAAGGAAG;
   NY-ESO-1 R: TCTGGCTAGCTCATCTTCTCTGTCCGCTA.
4) MAGE A4 F: ACGCTCGAGACAGAGGAGCACCAAGGAG;
   MAGE A4 R: TCTGGCTAGCATAGACTGAGGCATAAGGC.
5) Claude 18.2 F: ACGCTCGAGATGGACCAGTGGAGCACCC;
   Claude 18.2 R: TCTGGCTAGCTTAGGCGATGCACATCATC.
6) EGFR F: ACGCTCGAGATGCGACCCTCCGGGACGG;
   EGFR R: TCTGGCTAGCTTACATGAAGAGGCCGAT.
7) HER2 F: ATGGAGCTGGCGGCCTTGTGCC;
   HER2 R: TTAGATGAGGATCCCAAAGACCA.
8) ALK F: AAGCGGGGGCGGCAGCGGT;
   ALK R: TTTTAGTCATTACAAATAAC.
9) BTK F: ATGGCCGCAGTGATTCTGG;
   BTK R: GGATTCTTCATCCATGACA.
10) FGFR1 F: ATGTGGAGCTGGAAGTGCC;
   FGFR1 R: TCAGCGGCGTTTGAGTCCG.
11) VEGFR F: ACGCTCGAGATGCAGAGCAAGGTGCTG;
   VEGFR R: TCTGGCTAGCTCAGATGATGACAAGAAGT.
12) CDK4 F: ATGGCTACCTCTCGATATG;
   CDK4 R: TCACTCCGGATTACCTTCA.
13) CDK6 F: ATGGAGAAGGACGGCCTGT;
   CDK6 R: TCAGGCTGTATTCAGCTCC.
14) BRAF F: ATGGCGGCGCTGAGCGGTG;
   BRAF R: TCAGTGGACAGGAAACGCA.
15) MET F: ACGCTCGAGATGGAGTGCAAGGAGGCC;
   MET R: TCTGGCTAGCTTACAGCCACAGGAAGAAG.
16) KRAS G12C F: ATGACTGAATATAAACTTG;
   KRAS G12C R: TTACATTATAATGCATTTT.

The product was extracted after 1% agarose gel electrophoresis and sequenced by a sequencing company. The sequencing results are shown in Table 2.

**Table 2 Recombinant oncolytic viruses and small-molecule anticancer drugs in Preparation Examples 11-49**

| Preparati on Example | Protein | Antigen | | Small-molecule anticancer drug |
|---|---|---|---|---|
| | Mutated sites and amino acids after mutation | Type | Sequence | Type |
| 11 | M protein: N32S, N49D, M51R, H54Y, V221F, V225I, S226R. | EGFR | SEQ ID NO 25 | Mobocertinib |
| 12 | | ALK | SEQ ID NO 30 | Ceritinib |
| 13 | | BTK | SEQ ID NO 31 | Ibrutinib |
| 14 | | EGFR | SEQ ID NO 25 | Afatinib |
| 15 | | EGFR | SEQ ID NO 25 | Dacomitinib |
| 16 | | EGFR | SEQ ID NO 25 | Icotinib |
| 17 | | FGFR | SEQ ID NO 32 | Lenvatinib |
| 18 | G protein: V531, A141V, D172Y, K217E, D232G, V331A, V371E, G436D, T438S, F453L, T471I, Y487H. N protein: I14V, R155K, S353N. | FGFR | SEQ ID NO 32 | Pazopanib |
| 19 | P protein: R50K, V76A, D99E, L126S, L140S, H151Y, 1168M, K170E, Y189S, N237D. | FGFR | SEQ ID NO 32 | Anlotinib |
| 20 | L protein: S87P, I487T. | HER2 | SEQ ID NO 26 | Pyrotinib |
| 21 | | HER2 | SEQ ID NO 26 | Niraparib |
| 22 | | HER2 | SEQ ID NO 26 | Olaparib |
| 23 | | VEGFR | SEQ ID NO 33 | Regorafenib |
| 24 | | CDK4/6 | SEQ ID NO 34/35 | Palbociclib |
| 25 | | BRAF | SEQ ID NO 36 | Vemurafenib |
| 26 | | MET | SEQ ID NO 37 | Savolitinib |
| 27 | | KRAS G12C | SEQ ID NO 38 | Sotorasib |
| 28 | | MAGE A4 | SEQ ID NO 23 | Mobocertinib |
| 29 | | | | Ceritinib |
| 30 | | | | Ibrutinib |
| 31 | | | | Afatinib |
| 32 | | | | Dacomitinib |
| 33 | M protein: N32S, N49D, M51R, H54Y, V221F, V225I, S226R. | | | Icotinib |
| 34 | | | | Lenvatinib |
| 35 | G protein: V531, A141V, D172Y, K217E, D232G, V331A, V371E, G436D, T438S, F453L, T471I, Y487H. | | | Pazopanib |
| 36 | | | | Anlotinib |
| 37 | N protein: I14V, R155K, S353N. | | | Pyrotinib |
| 38 | P protein: R50K, V76A, D99E, L126S, L140S, H151Y, 1168M, K170E, Y189S, N237D. | | | Niraparib |
| 39 | | | | Olaparib |
| 40 | L protein: S87P, I487T. | | | Regorafenib |
| 41 | | | | Palbociclib |
| 42 | | | | Vemurafenib |
| 43 | | | | Savolitinib |
| 44 | | | | Sotorasib |
| 45 | | CD19 | SEQ ID NO 20 | Mobocertinib |
| 46 | M protein: N32S, N49D, M51R, H54Y, V221F, V225I, S226R. | CD22 | SEQ ID NO 21 | |
| 47 | G protein: V531, A141V, D172Y, K217E, D232G, V331A, V371E, G436D, T438S, F453L, T471I, Y487H. | NY-ESO -1 | SEQ ID NO 22 | |
| 48 | N protein: I14V, R155K, S353N. | Claude 18.2 | SEQ ID NO 24 | |
| | P protein: R50K, V76A, D99E, L126S, L140S, H151Y, 1168M, K170E, Y189S, N237D. L protein: S87P, I487T. | | | |
| 49 | | HER2 | SEQ ID NO 26 | |

### Preparation Examples 50-83

Preparation Examples 50-83 provide a method for treatment of a tumor by using a recombinant oncolytic virus in combination with a small-molecule anticancer drug, respectively.

In the method, the small-molecule anticancer drug and the recombinant oncolytic virus are used in combination to treat the tumor.

Said preparation examples are different from Preparation Example 11 in that the recombinant oncolytic virus further includes a cytokine, in addition to the M protein, the G protein, the N protein, the P protein, the L protein and the antigen. The mutated sites of the M protein, the G protein, the N protein, the P protein and the L protein are the same as the corresponding mutated sites in Preparation Example 2, and
the difference lies in the type of the cytokine and the type of the small-molecule anticancer drug. The mutated sites of the proteins, the type of the antigen, the type of the cytokine and the type of the small-molecule anticancer drug are shown in Table 3.

The construction method of the recombinant oncolytic viruses provided in said preparation examples is the same as the construction method in Preparation Example 2, except that:
Between the step (1) of construction of vector and the step (2) of virus rescue, the method further included the following step: insertion of an exogenous gene encoding the cytokine. For details, reference may be made to the step of insertion of an exogenous gene encoding the antigen. The antigen and the cytokine were inserted between the G and L proteins. With regard to the order of insertion of the antigen and the cytokine, the cytokine may be inserted first, then the antigen; or the antigen may be inserted first, then the cytokine. In the present application, the cytokinewas inserted first, then the antigen.

Step (3) further included sequencing of the cytokine. The viral genomic RNA was extracted using a Trizol kit, and reverse transcription was performed using random primers. The reverse transcribed cDNA was subjected to PCR using primers designed for the gene sequence of the cytokine.

The primer sequences designed for the gene sequence encoding the cytokines were:
IL12 F: CCCTCGAGATGTGGCCCCCTGGGT,
IL12 R: CGGCTAGCTTAACTGCAGGGCACAGATG.
IL-18 F: CCCTCGAGATGGCTGCTGAACCAGTAG,
IL-18 R: CGGCTAGCCTAGTCTTCGTTTTGAAC.

The product was extracted after 1% agarose gel electrophoresis and sequenced by a sequencing company. The sequencing results are shown in Table 3.

**Table 3 Recombinant oncolytic viruses and small-molecule anticancer drugs in Preparation Examples 50-83**

| Preparation Example | Mutated sites of proteins | Antigen/cytokine | | | Small-molecule anticancer drug |
|---|---|---|---|---|---|
| | | Type | Type | Sequence | Type |
| 50 | M protein: N32S, N49D, M51R, H54Y, V221F, V225I, S226R. | EGFR | IL-12 | SEQ ID NO 27, SEQ ID NO 28 | Mobocertinib |
| 51 | | ALK | | | Ceritinib |
| 52 | | BTK | | | Ibrutinib |
| 53 | | EGFR | | | Afatinib |
| 54 | G protein: V53I, A141V, D172Y, K217E, D232G, V331A, V371E, G436D, T438S, F453L, T471I, Y487H. | EGFR | | | Dacomitinib |
| 55 | | EGFR | | | Icotinib |
| 56 | | FGFR | | | Lenvatinib |
| 57 | | FGFR | | | Pazopanib |
| 58 | | FGFR | | | Anlotinib |
| 59 | N protein: I14V, R155K, S353N. | HER2 | | | Pyrotinib |
| 60 | | HER2 | | | Niraparib |
| 61 | P protein: R50K, V76A, D99E, L126S, L140S, H151Y, 1168M, K170E, Y189S, N237D. | HER2 | | | Olaparib |
| 62 | | VEGFR | | | Regorafenib |
| 63 | | CDK4/6 | | | Palbociclib |
| 64 | | BRAF | | | Vemurafenib |
| 65 | L protein: S87P, I487T. | MET | | | Savolitinib |
| 66 | | KRAS G12C | | | Sotorasib |
| 67 | M protein: N32S, N49D, M51R, H54Y, V221F, V225I, S226R. | EGFR | IL-18 | SEQ ID NO 29 | Mobocertinib |
| 68 | | ALK | | | Ceritinib |
| 69 | | BTK | | | Ibrutinib |
| 70 | | EGFR | | | Afatinib |
| 71 | G protein: V53I, A141V, D172Y, K217E, D232G, V331A, V371E, G436D, T438S, F453L, T471I, Y487H. | EGFR | | | Dacomitinib |
| 72 | | EGFR | | | Icotinib |
| 73 | | FGFR | | | Lenvatinib |
| 74 | | FGFR | | | Pazopanib |
| 75 | | FGFR | | | Anlotinib |
| 76 | N protein: I14V, R155K, S353N. | HER2 | | | Pyrotinib |
| 77 | | HER2 | | | Niraparib |
| 78 | P protein: R50K, V76A, D99E, L126S, L140S, H151Y, I168M, K170E, Y189S, N237D. | HER2 | | | Olaparib |
| 79 | | VEGFR | | | Regorafenib |
| 80 | | CDK4/6 | | | Palbociclib |
| 81 | | BRAF | | | Vemurafenib |
| 82 | L protein: S87P, I487T. | MET | | | Savolitinib |
| 83 | | KRAS G12C | | | Sotorasib |

### Preparation Examples 84-91

Preparation Examples 84-91 and 109-116 respectively provide a method for treating a tumor using a recombinant oncolytic virus.

The recombinant oncolytic viruses in said preparation examples were the recombinant oncolytic viruses prepared in Preparation Examples 2, 11, 28 and 45-49, respectively. See Table 4 for details. The difference lies in the type of the antigen expressed by the recombinant oncolytic viruses (e.g., Preparation Examples 84-90 and 109-116) or in the recombinant oncolytic viruses without expressing the antigen (e.g., Preparation Example 91).

**Table 4 Recombinant oncolytic viruses in Preparation Examples 71-78**

| Preparation Example | M protein | Antigen | |
|---|---|---|---|
| | Mutated sites and amino acids after mutation | Type | Sequence |
| 84 | M protein: N32S, N49D, M51R, H54Y, V221F, V225I, S226R. | CD19 | SEQ ID NO 20 |
| 85 | | CD22 | SEQ ID NO 21 |
| 86 | | NY-ESO-1 | SEQ ID NO 22 |
| 87 | | MAGE A4 | SEQ ID NO 23 |
| 88 | | Claude 18.2 | SEQ ID NO 24 |
| 89 | G protein: V53I, A141V, D172Y, K217E, D232G, V331A, V371E, G436D, T438S, F453L, T471I, Y487H. | EGFR | SEQ ID NO 25 |
| 90 | | HER2 | SEQ ID NO 26 |
| 91 | | - | - |
| 109 | | ALK | SEQ ID NO 30 |
| 110 | N protein: I14V, R155K, S353N. | BTK | SEQ ID NO 31 |
| 111 | | FGFR | SEQ ID NO 32 |
| 112 | P protein: R50K, V76A, D99E, L126S, L140S, H151Y, 1168M, K170E, Y189S, N237D. | VEGFR | SEQ ID NO 33 |
| 113 | | CDK4/6 | SEQ ID NO 34/35 |
| 114 | | BRAF | SEQ ID NO 36 |
| 115 | L protein: S87P, I487T. | MET | SEQ ID NO 37 |
| 116 | | KRAS G12C | SEQ ID NO 38 |
| Note: "-" means no antigen inserted | | | |

### Preparation Examples 92-108

Preparation Examples 92-108 provide a method for treating the tumor using a small-molecule anticancer drug, respectively.

The small-molecule anticancer drug in said preparation examples were the small-molecule anticancer drugs in Preparation Examples 11-27, respectively. See Table 5 for details. The difference lies in the type of the small-molecule anticancer drug.

**Table 5 Small-molecule anticancer drugs in Preparation Examples 92-108**

| Preparation Example | Small-molecule anticancer drug |
|---|---|
| | Type |
| 92 | Mobocertinib |
| 93 | Ceritinib |
| 94 | Ibrutinib |
| 95 | Afatinib |
| 96 | Dacomitinib |
| 97 | Icotinib |
| 98 | Lenvatinib |
| 99 | Pazopanib |
| 100 | Anlotinib |
| 101 | Pyrotinib |
| 102 | Niraparib |
| 103 | Olaparib |
| 104 | Regorafenib |
| 105 | Palbociclib |
| 106 | Vemurafenib |
| 107 | Savolitinib |
| 108 | Sotorasib |

### EXAMPLE

In this example, animal experiments were performed using the methods for treatment of a tumor by using a recombinant oncolytic virus and/or a small molecule anticancer drug provided in Preparation Examples 1-116.

Balb/c mice were prepared and inoculated with H22 cells (mouse liver cancer cells) at a concentration of 5×10⁵/0.1 mL/animal. When the average tumor size of mice reached 50 mm³, the mice were grouped, and that day was denoted as Day 0. The drug was administered on the day of grouping. The mice were weighed three times a week and observed once a day. Changes in the body weight (g) and tumor volume (mm³) of mice were recorded during the experiments.

If the following conditions occurred, the mice were euthanized: 1. the tumor volume reached 2000 mm³; 2. the mice lost more than 20% body weight; 3. the tumor surface had ulceration; 4. Paralysis or something like that occurred in the mice.

The recombinant oncolytic virus could be administered in many different ways. According to the present application, in case of wild-type oncolytic virus and recombinant oncolytic viruses, the administration route was intratumoral injection (IT) and/or intravenous injection (IV), with a dose of 3e8 PFU/animal. The administration volume of IT was 2.5 ml/kg. The administration volume of IV was 5ml/kg. The administration frequency was once every 2 days for 6 consecutive times (Q2D *6).

In case of small-molecule anticancer drugs, the administration route was oral administration (PO), and the dose was related to the type of small-molecule anticancer drug. The administration volume was 10 ml/kg, and the administration frequency was once a day for 12 consecutive days (QD*12); alternatively, the administration frequency was twice a day for 12 consecutive days (BID*12).

In case of Vehicle Control, the Vehicle (10% DMSO +40% PEG300 + 5% Tween-80+ 45% saline) was used instead of small-molecule anticancer drugs. The administration route was oral administration (PO), the dose was N/A, the administration volume was 10 ml/kg, and the administration frequency was once a day for 12 consecutive days (QD*12).

### 1. Animal experiments on the small-molecule anticancer drug Mobocertinib

Animal experiments were performed using the methods of treatment of tumors using a recombinant oncolytic virus and/or a small-molecule anticancer drug provided in Preparation Examples 1-10, 11, 28, 45-49, 50, 67, 84-91, and 92. Details of the experiments are shown in Table 6.

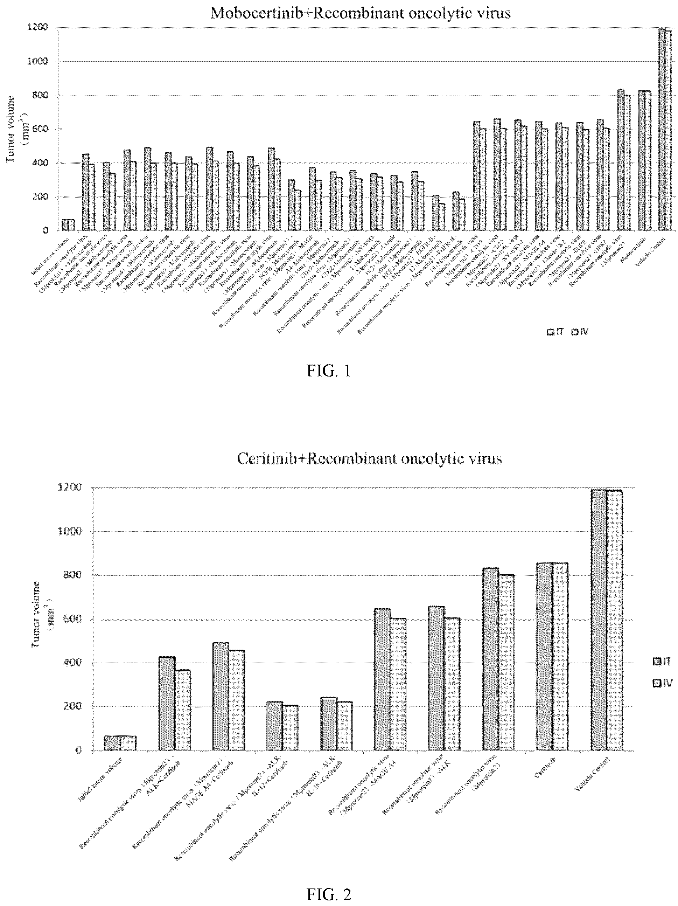

The test results are shown in FIG. 1.

**Table 6 Animal experiments on the small-molecule anticancer drug Mobocertinib**

| Group | Correspon ding Preparatio n Example | Corresponding method | Numb er of anima ls | Dose | Admini stration volume (mL/kg) | Admini stration frequen cy | Admini stration route | Admin istratio n route |
|---|---|---|---|---|---|---|---|---|
| 1 | 1 | Recombinant oncolytic virus (M protein 1)+Mobocertinib | 10 | 3e8 PFU/ani | 0.05+10 | Q2D*6 + | IT+PO | IV+PO |
| 2 | 2 | Recombinant oncolytic virus (M protein 2)+Mobocertinib | 10 | mal+30 mg/kg | | QD*12 | | |
| 3 | 3 | Recombinant oncolytic virus (M protein 3)+Mobocertinib | 10 | | | | | |
| 4 | 4 | Recombinant oncolytic virus (M protein 4)+Mobocertinib | 10 | | | | | |
| 5 | 5 | Recombinant oncolytic virus (M protein 5)+Mobocertinib | 10 | | | | | |
| 6 | 6 | Recombinant oncolytic virus (M protein 6)+Mobocertinib | 10 | | | | | |
| 7 | 7 | Recombinant oncolytic virus (M protein 7)+Mobocertinib | 10 | | | | | |
| 8 | 8 | Recombinant oncolytic virus (M protein 8)+Mobocertinib | 10 | | | | | |
| 9 | 9 | Recombinant oncolytic virus (M protein 9)+Mobocertinib | 10 | | | | | |
| 10 | 10 | Recombinant oncolytic virus (M protein 10)+Mobocertinib | 10 | | | | | |
| 11 | 11 | Recombinant oncolytic virus (M protein 2)-EGFR+Mobocertinib | 10 | | | | | |
| 12 | 28 | Recombinant oncolytic virus (M protein 2)-MAGE A4+Mobocertinib | 10 | | | | | |
| 13 | 45 | Recombinant oncolytic virus (M protein 2)-CD19+Mobocertinib | 10 | | | | | |
| 14 | 46 | Recombinant oncolytic virus (M protein 2)-CD22+Mobocertinib | 10 | | | | | |
| 15 | 47 | Recombinant oncolytic virus (M protein 2)-NY-ESO-1+Mobocertinib | 10 | | | | | |
| 16 | 48 | Recombinant oncolytic virus (M protein 2)-Claude 18.2+Mobocertinib | 10 | | | | | |
| 17 | 49 | Recombinant oncolytic virus (M protein 2)-HER2+Mobocertinib | 10 | | | | | |
| 18 | *50* | Recombinant oncolytic virus (M protein 2)-EGFR-IL-12+Mobocertinib | 10 | | | | | |
| 19 | 67 | Recombinant oncolytic virus (M protein 2)-EGFR-IL-18+Mobocertinib | 10 | | | | | |
| 20 | 84 | Recombinant oncolytic virus (M protein 2)-CD19 | 10 | | | | | |
| 21 | 85 | Recombinant oncolytic virus (M protein 2)-CD22 | 10 | | | | | |
| 22 | 86 | Recombinant oncolytic virus (M protein 2)-NY-ESO-1 | 10 | | | | | |
| 23 | 87 | Recombinant oncolytic virus (M protein 2)-MAGE A4 | 10 | | | | | |
| 24 | 88 | Recombinant oncolytic virus (M protein 2)-Claude 18.2 | 10 | | | | | |
| 25 | 89 | Recombinant oncolytic virus (M protein 2)-EGFR | 10 | | | | | |
| 26 | 90 | Recombinant oncolytic virus (M protein 2)-HER2 | 10 | | | | | |
| 27 | 91 | Recombinant oncolytic virus (M protein 2) | 10 | 3e8 PFU/ani mal | *0.05* | Q2D*6 | IT | IV |
| 28 | 92 | Mobocertinib | 10 | 30mg/k g | 10 | QD*12 | PO | PO |
| 29 | Vehicle Control | | 10 | N/A | 10 | QD*12 | PO | PO |

### 2. Animal experiments on the small-molecule anticancer drug Ceritinib

Animal experiments were performedusing the methods of treatment of tumors using a recombinant oncolytic virus and/or a small-molecule anticancer drug provided in Preparation Examples 12, 29, 51, 68, 87, 109, 91 and 93. Details of the experiments are shown in Table 7.

The test results are shown in FIG. 2.

**Table 7 Animal experiments on the small-molecule anticancer drug Ceritinib**

| Grou p | Correspond ing Preparation Example | Corresponding method | Numb er of anima Is | Dose | Administrat ion volume (mL/kg) | Administrat ion frequency | Administrat ion route | Administrat ion route |
|---|---|---|---|---|---|---|---|---|
| 1 | 12 | Recombinant oncolytic virus (M protein 2)-ALK+Ceritinib | 10 | 3e8 PFU/animal +70 mg/kg | 0.05+10 | Q2D*6 + QD*12 | IT+PO | IV+PO |
| 2 | 29 | Recombinant oncolytic virus (M protein 2)-MAGE A4+Ceritinib | 10 | | | | | |
| 3 | 51 | Recombinant oncolytic virus (M protein 2)-ALK-IL-12+Cer itinib | 10 | | | | | |
| 4 | 68 | Recombinant oncolytic virus (M protein 2)-ALK-IL-18+Cer itinib | 10 | | | | | |
| 5 | 87 | Recombinant oncolytic virus (M protein 2)-MAGE A4 | 10 | | | | | |
| 6 | 109 | Recombinant oncolytic virus (M protein 2)-ALK | 10 | | | | | |
| 7 | 91 | Recombinant oncolytic virus (M protein 2) | 10 | 3e8 PFU/animal | 0.05 | Q2D*6 | IT | IV |
| 8 | 92 | Ceritinib | 10 | 70mg/kg | 10 | QD*12 | PO | PO |
| 9 | Vehicle Control | | 10 | N/A | 10 | QD*12 | PO | PO |

### 3. Animal experiments on the small-molecule anticancer drug Ibrutinib

Animal experiments were performed using the methods of treatment of tumors using a recombinant oncolytic virus and/or a small-molecule anticancer drug provided in Preparation Examples 13, 30, 52, 69, 87, 110, 91 and 94. Details of the experiments are shown in Table 8.

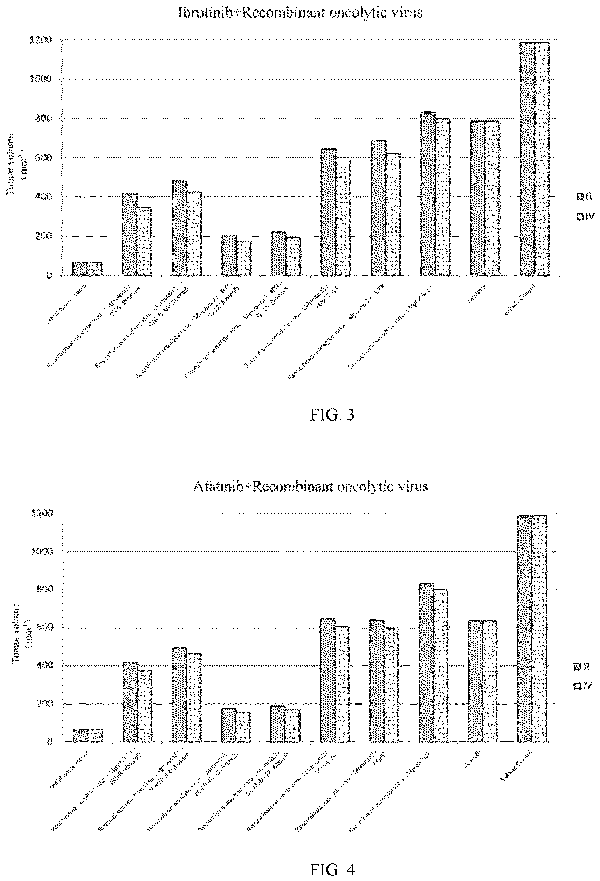

The test results are shown in FIG. 3.

**Table 8 Animal experiments on the small-molecule anticancer drug Ibrutinib**

| Gro up | Correspond ing Preparation Example | Corresponding method | Numb er of anima ls | Dose | Administrat ion volume (mL/kg) | Administrat ion frequency | Administrat ion route | Administrat ion route |
|---|---|---|---|---|---|---|---|---|
| 1 | 13 | Recombinant oncolytic virus (M protein 2)-BTK+Ibrutinib | 10 | 3e8 PFU/animal +75 mg/kg | 0.05+10 | Q2D*6 + QD*12 | IT+PO | IV+PO |
| 2 | 30 | Recombinant oncolytic virus (M protein 2)-MAGE A4+Ibrutinib | 10 | | | | | |
| 3 | 52 | Recombinant oncolytic virus (M protein 2)-BTK-IL-12+Ibr utinib | 10 | | | | | |
| 4 | 69 | Recombinant oncolytic virus (M protein 2)-BTK-IL-18+Ibr utinib | 10 | | | | | |
| 5 | 87 | Recombinant oncolytic virus (M protein 2)-MAGE A4 | 10 | | | | | |
| 6 | 110 | Recombinant oncolytic virus (M protein 2)-BTK | 10 | | | | | |
| 7 | 91 | Recombinant oncolytic virus (M protein 2) | 10 | 3e8 PFU/animal | 0.05 | Q2D*6 | IT | IV |
| 8 | 92 | Ibrutinib | 10 | 75mg/kg | 10 | QD*12 | PO | PO |
| 9 | Vehicle Control | | 10 | N/A | 10 | QD*12 | PO | PO |

### 4. Animal experiments on the small-molecule anticancer drug Afatinib

Animal experiments were preformed using the methods of treatment of tumors using a recombinant oncolytic virus and/or a small-molecule anticancer drug provided in Preparation Examples 14, 31, 53, 70, 87, 89, 91 and 95. Details of the experiments are shown in Table 9.

The test results are shown in FIG. 4.

**Table 9 Animal experiments on the small-molecule anticancer drug Afatinib**

| Grou p | Correspond ing Preparation Example | Corresponding method | Numb er of animal s | Dose | Administrat ion volume (mL/kg) | Administrat ion frequency | Administrat ion route | Administrat ion route |
|---|---|---|---|---|---|---|---|---|
| 1 | 14 | Recombinant oncolytic virus (M protein 2)-EGFR+Afatinib | 10 | 3e8 PFU/animal+6m g/kg | 0.05+10 | Q2D*6 + QD*12 | IT+PO | IV+PO |
| 2 | 31 | Recombinant oncolytic virus (M protein 2)-MAGE A4+Afatinib | 10 | | | | | |
| 3 | 53 | Recombinant oncolytic virus (M protein 2)-EGFR-IL-12+Afa tinib | 10 | | | | | |
| 4 | 70 | Recombinant oncolytic virus (M protein 2)-EGFR-IL-18+Afa tinib | 10 | | | | | |
| 5 | 87 | Recombinant oncolytic virus (M protein 2)-MAGE A4 | 10 | | | | | |
| 6 | 89 | Recombinant oncolytic virus (M protein 2)-EGFR | 10 | | | | | |
| 7 | 91 | Recombinant oncolytic virus (M protein 2) | 10 | 3e8 PFU/animal | 0.05 | Q2D*6 | IT | IV |
| 8 | 92 | Afatinib | 10 | 6mg/kg | 10 | QD*12 | PO | PO |
| 9 | Vehicle Control | | 10 | N/A | 10 | QD*12 | PO | PO |

### 5. Animal experiments on the small-molecule anticancer drug Dacomitinib

Animal experiments were performed using the methods of treatment of tumors using a recombinant oncolytic virus and/or a small-molecule anticancer drug provided in Preparation Examples 15, 32, 54, 71, 87, 89, 91 and 96. Details of the experiments are shown in Table 10.

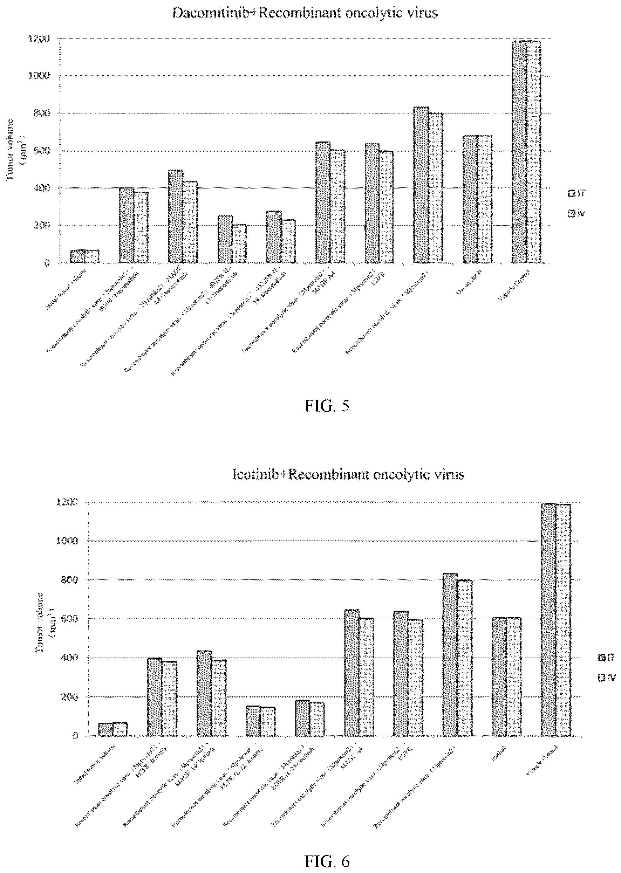

The test results are shown in FIG. 5.

**Table 10 Animal experiments on the small-molecule anticancer drug Dacomitinib**

| Grou p | Correspondi ng Preparation Example | Corresponding method | Numb er of animal s | Dose | Administrati on volume (mL/kg) | Administrati on frequency | Administrati on route | Administrati on route |
|---|---|---|---|---|---|---|---|---|
| 1 | 15 | Recombinant oncolytic virus (M protein 2)-EGFR+Dacomitinib | 10 | 3e8 PFU/animal+ 6.5 mg/kg | 0.05+10 | Q2D*6 + QD*12 | IT+PO | IV+PO |
| 2 | 32 | Recombinant oncolytic virus (M protein 2)-MAGE A4+Dacomitinib | 10 | | | | | |
| 3 | 54 | Recombinant oncolytic virus (M protein 2)-EGFR-IL-12+Dacomi tinib | 10 | | | | | |
| 4 | 70 | Recombinant oncolytic virus (M protein 2)-EGFR-IL-18+Dacomi tinib | 10 | | | | | |
| 5 | 87 | Recombinant oncolytic virus (M protein 2)-MAGE A4 | 10 | | | | | |
| 6 | 89 | Recombinant oncolytic virus (M protein 2)-EGFR | 10 | | | | | |
| 7 | 91 | Recombinant oncolytic virus (M protein 2) | 10 | 3e8 PFU/animal | 0.05 | Q2D*6 | IT | IV |
| 8 | 92 | Dacomitinib | 10 | 6.5mg/kg | 10 | QD*12 | PO | PO |
| 9 | Vehicle Control | | 10 | N/A | 10 | QD*12 | PO | PO |

### 6. Animal experiments on the small-molecule anticancer drug Icotinib

Animal experiments were performed using the methods of treatment of tumors using a recombinant oncolytic virus and/or a small-molecule anticancer drug provided in Preparation Examples 16, 33, 55, 72, 87, 89, 91 and 97. Details of the experiments are shown in Table 11.

The test results are shown in FIG. 6.

**Table 11 Animal experiments on the small-molecule anticancer drug Icotinib**

| Grou p | Correspondi ng Preparation Example | Corresponding method | Numb er of animal s | Dose | Administrati on volume (mL/kg) | Administrati on frequency | Administrati on route | Administrati on route |
|---|---|---|---|---|---|---|---|---|
| 1 | 16 | Recombinant oncolytic virus (M protein 2)-EGFR+Icotinib | 10 | 3e8 PFU/animal+ 55 mg/kg | 0.05+10 | Q2D*6 + QD*12 | IT+PO | IV+PO |
| 2 | 33 | Recombinant oncolytic virus (M protein 2)-MAGE A4+Icotinib | 10 | | | | | |
| 3 | 55 | Recombinant oncolytic virus (M protein 2)-EGFR-IL-12+Icot inib | 10 | | | | | |
| 4 | 72 | Recombinant oncolytic virus (M protein 2)-EGFR-IL-18+Icot inib | 10 | | | | | |
| 5 | 87 | Recombinant oncolytic virus (M protein 2)-MAGE A4 | 10 | | | | | |
| 6 | 89 | Recombinant oncolytic virus (M protein 2)-EGFR | 10 | | | | | |
| 7 | 91 | Recombinant oncolytic virus (M protein 2) | 10 | 3e8 PFU/animal | 0.05 | Q2D*6 | IT | IV |
| 8 | 92 | Icotinib | 10 | 55mg/kg | 10 | QD*12 | PO | PO |
| 9 | Vehicle Control | | 10 | N/A | 10 | QD*12 | PO | PO |

### 7. Animal experiments on the small-molecule anticancer drug Lenvatinib

Animal experiments were performed using the methods of treatment of tumors using a recombinant oncolytic virus and/or a small-molecule anticancer drug provided in Preparation Examples 17, 34, 56, 73, 87, 111, 91 and 98. Details of the experiments are shown in Table 12.

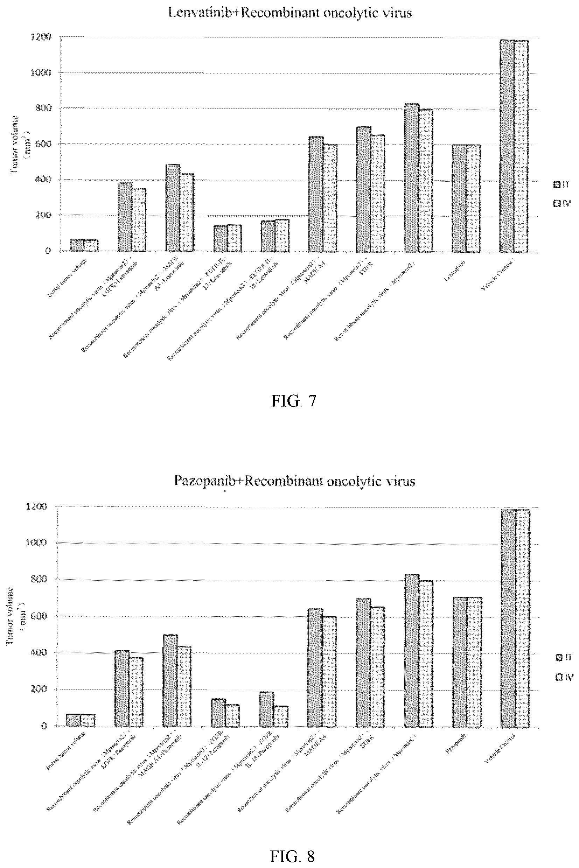

The test results are shown in FIG. 7.

**Table 12 Animal experiments on the small-molecule anticancer drug Lenvatinib**

| Grou p | Correspondi ng Preparation Example | Corresponding method | Numb er of animal s | Dose | Administrati on volume (mL/kg) | Administrati on frequency | Administrati on route | Administrati on route |
|---|---|---|---|---|---|---|---|---|
| 1 | 17 | Recombinant oncolytic virus (M protein 2)-FGFR+Lenvatinib | 10 | 3e8 PFU/animal +2 mg/kg | 0.05+10 | Q2D*6 + QD*12 | IT+PO | IV+PO |
| 2 | 34 | Recombinant oncolytic virus (M protein 2)-MAGE A4+Lenvatinib | 10 | | | | | |
| 3 | 56 | Recombinant oncolytic virus (M protein 2)-FGFR-IL-12+Lenva tinib | 10 | | | | | |
| 4 | 73 | Recombinant oncolytic virus (M protein 2)-FGFR-IL-18+Lenva tinib | 10 | | | | | |
| 5 | 87 | Recombinant oncolytic virus (M protein 2)-MAGE A4 | 10 | | | | | |
| 6 | 111 | Recombinant oncolytic virus (M protein 2)-FGFR | 10 | | | | | |
| 7 | 91 | Recombinant oncolytic virus (M protein 2) | 10 | 3e8 PFU/animal | 0.05 | Q2D*6 | IT | IV |
| 8 | 92 | Lenvatinib | 10 | 2mg/kg | 10 | QD*12 | PO | PO |
| 9 | Vehicle Control | | 10 | N/A | 10 | QD*12 | PO | PO |

### 8. Animal experiments on the small-molecule anticancer drug Pazopanib

Animal experiments were performed using the methods of treatment of tumors using a recombinant oncolytic virus and/or a small-molecule anticancer drug provided in Preparation Examples 18, 35, 57, 74, 87, 111, 91 and 99. Details of the trials are shown in Table 13.

The test results are shown in FIG. 8.

**Table 13 Animal experiments on the small-molecule anticancer drug Pazopanib**

| Grou p | Correspondi ng Preparation Example | Corresponding method | Numb er of animal s | Dose | Administrati on volume (mL/kg) | Administrati on frequency | Administrati on route | Administrati on route |
|---|---|---|---|---|---|---|---|---|
| 1 | 18 | Recombinant oncolytic virus (M protein 2)-FGFR+Pazopanib | 10 | 3e8 PFU/animal +30 mg/kg | 0.05+10 | Q2D*6 + QD*12 | IT+PO | IV+PO |
| 2 | 35 | Recombinant oncolytic virus (M protein 2)-MAGE A4+Pazopanib | 10 | | | | | |
| 3 | 57 | Recombinant oncolytic virus (M protein 2)-FGFR-IL-12+Pazo panib | 10 | | | | | |
| 4 | 74 | Recombinant oncolytic virus (M protein 2)-FGFR-IL-18+Pazo panib | 10 | | | | | |
| 5 | 87 | Recombinant oncolytic virus (M protein 2)-MAGE A4 | 10 | | | | | |
| 6 | 111 | Recombinant oncolytic virus (M protein 2)-FGFR | 10 | | | | | |
| 7 | 91 | Recombinant oncolytic virus (M protein 2) | 10 | 3e8 PFU/animal | 0.05 | Q2D*6 | IT | IV |
| 8 | 92 | Pazopanib | 10 | 30mg/kg | 10 | QD*12 | PO | PO |
| 9 | Vehicle Control | | 10 | N/A | 10 | QD*12 | PO | PO |

### 9. Animal experiments on the small-molecule anticancer drug Anlotinib

Animal experiments were performed using the methods of treatment of tumors using a recombinant oncolytic virus and/or a small-molecule anticancer drug provided in Preparation Examples 19, 36, 58, 75, 87, 111, 91 and 100. Details of the experiments are shown in Table 14.

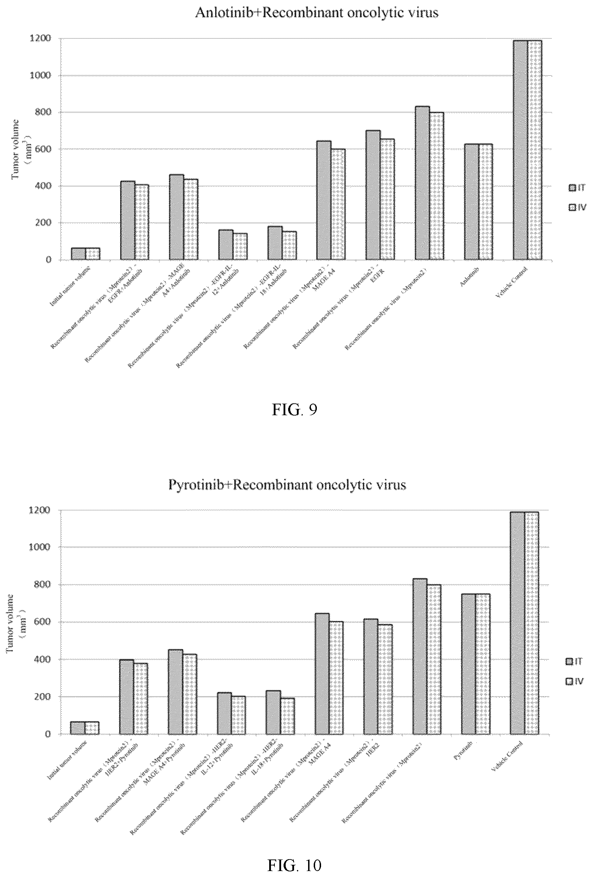

The test results are shown in FIG. 9.

**Table 14 Animal experiments on the small-molecule anticancer drug Anlotinib**

| Grou p | Correspondi ng Preparation Example | Corresponding method | Numb er of animal s | Dose | Administrati on volume (mL/kg) | Administrati on frequency | Administrati on route | Administrati on route |
|---|---|---|---|---|---|---|---|---|
| 1 | 19 | Recombinant oncolytic virus (M protein 2)-FGFR+Anlotinib | 10 | 3e8 PFU/animal +2 mg/kg | 0.05+10 | Q2D*6 + QD*12 | IT+PO | IV+PO |
| 2 | 36 | Recombinant oncolytic virus (M protein 2)-MAGE A4+Anlotinib | 10 | | | | | |
| 3 | 58 | Recombinant oncolytic virus (M protein 2)-FGFR-IL-12+Anlot inib | 10 | | | | | |
| 4 | 75 | Recombinant oncolytic virus (M protein 2)-FGFR-IL-18+Anlot inib | 10 | | | | | |
| 5 | 87 | Recombinant oncolytic virus (M protein 2)-MAGE A4 | 10 | | | | | |
| 6 | 111 | Recombinant oncolytic virus (M protein 2)-FGFR | 10 | | | | | |
| 7 | 91 | Recombinant oncolytic virus (M protein 2) | 10 | 3e8 PFU/animal | 0.05 | Q2D*6 | IT | IV |
| 8 | 92 | Anlotinib | 10 | 2mg/kg | 10 | QD*12 | PO | PO |
| 9 | Vehicle Control | | 10 | N/A | 10 | QD*12 | PO | PO |

### 10. Animal experiments on the small-molecule anticancer drug Pyrotinib

Animal experiments were performed using the methods of treatment of tumors using a recombinant oncolytic virus and/or a small-molecule anticancer drug provided in Preparation Examples 20, 37, 59, 76, 87, 90, 91 and 101. Details of the experiments are shown in Table 15.

The test results are shown in FIG. 10.

**Table 15 Animal experiments on the small-molecule anticancer drug Pyrotinib**

| Grou p | Correspondi ng Preparation Example | Corresponding method | Numb er of animal s | Dose | Administrati on volume (mL/kg) | Administrati on frequency | Administrati on route | Administrati on route |
|---|---|---|---|---|---|---|---|---|
| 1 | 20 | Recombinant oncolytic virus (M protein 2)-HER2+Pyrotinib | 10 | 3e8 PFU/animal +60 mg/kg | 0.05+10 | Q2D*6 + QD*12 | IT+PO | IV+PO |
| 2 | 37 | Recombinant oncolytic virus (M protein 2)-MAGE A4+Pyrotinib | 10 | | | | | |
| 3 | 59 | Recombinant oncolytic virus (M protein 2)-HER2-IL-12+Pyro tinib | 10 | | | | | |
| 4 | 76 | Recombinant oncolytic virus (M protein 2)-HER2-IL-18+Pyro tinib | 10 | | | | | |
| 5 | 87 | Recombinant oncolytic virus (M protein 2)-MAGE A4 | 10 | | | | | |
| 6 | 90 | Recombinant oncolytic virus (M protein 2)-HER2 | 10 | | | | | |
| 7 | 91 | Recombinant oncolytic virus (M protein 2) | 10 | 3e8 PFU/animal | 0.05 | Q2D*6 | IT | IV |
| 8 | 92 | Pyrotinib | 10 | 60mg/kg | 10 | QD*12 | PO | PO |
| 9 | Vehicle Control | | 10 | N/A | 10 | QD*12 | PO | PO |

### 11. Animal experiments on the small-molecule anticancer drug Niraparib

Animal experiments were performed using the methods of treatment of tumors using a recombinant oncolytic virus and/or a small-molecule anticancer drug provided in Preparation Examples 21, 38, 60, 77, 87, 90, 91 and 102. Details of the experiments are shown in Table 16.

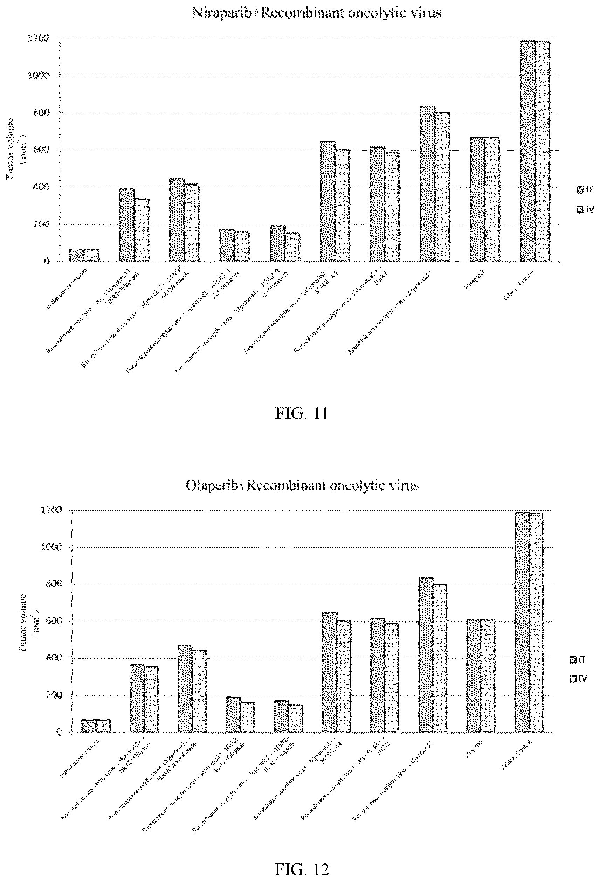

The test results are shown in FIG. 11.

**Table 16 Animal experiments on the small-molecule anticancer drug Niraparib**

| Group | Correspond ing Preparation Example | Corresponding method | Numb er of anima Is | Dose | Administrat ion volume (mL/kg) | Administrat ion frequency | Administrat ion route | Administrat ion route |
|---|---|---|---|---|---|---|---|---|
| 1 | 21 | Recombinant oncolytic virus (M protein 2)-HER2+Niraparib | 10 | 3e8 PFU/animal +45 mg/kg | 0.05+10 | Q2D*6 + QD*12 | IT+PO | IV+PO |
| 2 | 39 | Recombinant oncolytic virus (M protein 2)-MAGE A4+Niraparib | 10 | | | | | |
| 3 | 60 | Recombinant oncolytic virus (M protein 2)-HER2-IL-12+Nira parib | 10 | | | | | |
| 4 | 77 | Recombinant oncolytic virus (M protein 2)-HER2-IL-18+Nira parib | 10 | | | | | |
| 5 | 87 | Recombinant oncolytic virus (M protein 2)-MAGE A4 | 10 | | | | | |
| 6 | 90 | Recombinant oncolytic virus (M protein 2)-HER2 | 10 | | | | | |
| 7 | 91 | Recombinant oncolytic virus (M protein 2) | 10 | 3e8 PFU/animal | 0.05 | Q2D*6 | IT | IV |
| 8 | 92 | Niraparib | 10 | 45mg/kg | 10 | QD*12 | PO | PO |
| 9 | Vehicle Control | | 10 | N/A | 10 | QD*12 | PO | PO |

### 12. Animal experiments on the small-molecule anticancer drug Olaparib

Animal experiments were performed using the methods of treatment of tumors using a recombinant oncolytic virus and/or a small-molecule anticancer drug provided in Preparation Examples 22, 39, 61, 78, 87, 90, 91 and 103. Details of the experiments are shown in Table 17.

The test results are shown in FIG. 12.

**Table 17 Animal experiments on the small-molecule anticancer drug Olaparib**

| Group | Correspon ding Preparatio n Example | Corresponding method | Numb er of anima Is | Dose | Administrat ion volume (mL/kg) | Administr ation frequency | Admin istratio n route | Admi nistrat ion route |
|---|---|---|---|---|---|---|---|---|
| 1 | 22 | Recombinant oncolytic virus (M protein 2)-HER2+Olaparib | 10 | 3e8 PFU/ani | 0.05+10 | Q2D*6 + | IT+PO | IV+P O |
| 2 | 40 | Recombinant oncolytic virus (M protein 2)-MAGE A4+Olaparib | 10 | mal+45 mg/kg | | BID*12 | | |
| 3 | 61 | Recombinant oncolytic virus (M protein 2)-HER2-IL-12+Olaparib | 10 | | | | | |
| 4 | 78 | Recombinant oncolytic virus (M protein 2)-HER2-IL-18+Olaparib | 10 | | | | | |
| 5 | 87 | Recombinant oncolytic virus (M protein 2)-MAGE A4 | 10 | | | | | |
| 6 | 90 | Recombinant oncolytic virus (M protein 2)-HER2 | 10 | | | | | |
| 7 | 91 | Recombinant oncolytic virus (M protein 2) | 10 | 3e8 PFU/ani mal | 0.05 | Q2D*6 | IT | IV |
| 8 | 92 | Olaparib | 10 | 45mg/k g | 10 | BID*12 | PO | PO |
| 9 | Vehicle Control | | 10 | N/A | 10 | BID*12 | PO | PO |

### 13. Animal experiments on the small-molecule anticancer drug Regorafenib

Animal experiments were performed using the methods of treatment of tumors using a recombinant oncolytic virus and/or a small-molecule anticancer drug provided in Preparation Examples 23, 40, 62, 79, 87, 112, 91 and 104. Details of the experiments are shown in Table 18.

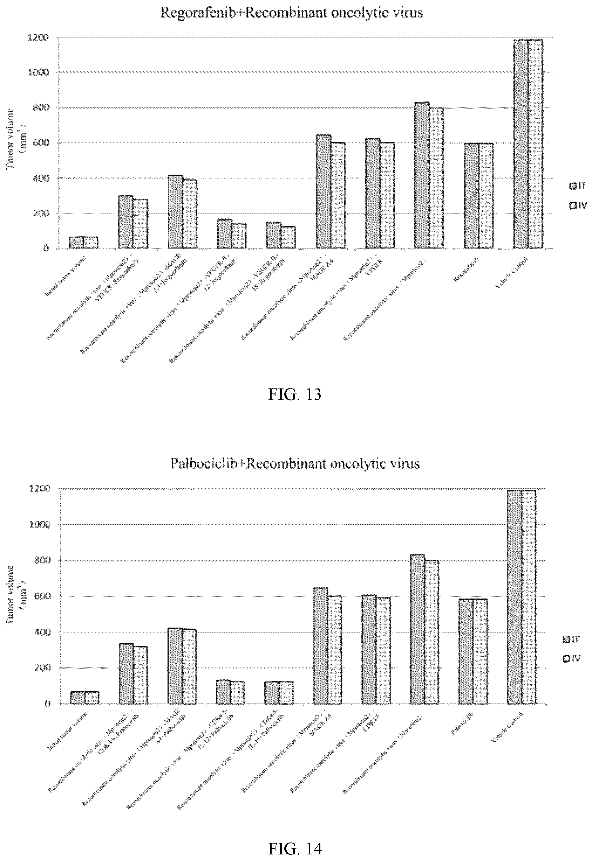

The test results are shown in FIG. 13.

**Table 18 Animal experiments on the small-molecule anticancer drug Regorafenib**

| Gro up | Correspond ing Preparation Example | Corresponding method | Numb er of anima ls | Dose | Administrat ion volume (mL/kg) | Administrat ion frequency | Administrat ion route | Administrat ion route |
|---|---|---|---|---|---|---|---|---|
| 1 | 23 | Recombinant oncolytic virus (M protein 2)-VEGFR+Regorafeni b | 10 | 3e8 PFU/animal +15 mg/kg | 0.05+10 | Q2D*6 + QD*12 | IT+PO | IV+PO |
| 2 | 40 | Recombinant oncolytic virus (M protein 2)-MAGE A4+Regorafenib | 10 | | | | | |
| 3 | 62 | Recombinant oncolytic virus (M protein 2)-VEGFR-IL-12+Rego rafenib | 10 | | | | | |
| 4 | 79 | Recombinant oncolytic virus (M protein 2)-VEGFR-IL-18+Rego rafenib | 10 | | | | | |
| 5 | 87 | Recombinant oncolytic virus (M protein 2)-MAGE A4 | 10 | | | | | |
| 6 | 112 | Recombinant oncolytic virus (M protein 2)-VEGFR | 10 | | | | | |
| 7 | 91 | Recombinant oncolytic virus (M protein 2) | 10 | 3e8 PFU/animal | 0.05 | Q2D*6 | IT | IV |
| 8 | 92 | Regorafenib | 10 | 15mg/kg | 10 | QD*12 | PO | PO |
| 9 | Vehicle Control | | 10 | N/A | 10 | QD*12 | PO | PO |

### 14. Animal experiments on the small-molecule anticancer drug Palbociclib

Animal experiments were performed using the methods of treatment of tumors using a recombinant oncolytic virus and/or a small-molecule anticancer drug provided in Preparation Examples 24, 41, 63, 80, 87, 113, 91 and 105. Details of the experiments are shown in Table 19.

The test results are shown in FIG. 14.

**Table 19 Animal experiments on the small-molecule anticancer drug Palbociclib**

| Gro up | Correspon ding Preparation Example | Corresponding method | Numb er of anima Is | Dose | Administra tion volume (mL/kg) | Administra tion frequency | Administra tion route | Administra tion route |
|---|---|---|---|---|---|---|---|---|
| 1 | 24 | Recombinant oncolytic virus (M protein 2)-CDK4/6+Palbocicli b | 10 | 3e8 PFU/animal+15 mg/kg | 0.05+10 | Q2D*6 + QD*12 | IT+PO | IV+PO |
| 2 | 41 | Recombinant oncolytic virus (M protein 2)-MAGE A4+Palbociclib | 10 | | | | | |
| 3 | 63 | Recombinant oncolytic virus (M protein 2)-CDK4/6-IL-12+Palb ociclib | 10 | | | | | |
| 4 | 80 | Recombinant oncolytic virus (M protein 2)-CDK4/6-IL-18+Palb ociclib | 10 | | | | | |
| 5 | 87 | Recombinant oncolytic virus (M protein 2)-MAGE A4 | 10 | | | | | |
| 6 | 113 | Recombinant oncolytic virus (M protein 2)-CDK4/6 | 10 | | | | | |
| 7 | 91 | Recombinant oncolytic virus (M protein 2) | 10 | 3e8 PFU/animal | *0.05* | Q2D*6 | IT | IV |
| 8 | 92 | Palbociclib | 10 | 15mg/kg | 10 | QD*12 | PO | PO |
| 9 | Vehicle Control | | 10 | N/A | 10 | QD*12 | PO | PO |

### 15. Animal experiments on the small-molecule anticancer drug Vemurafenib

Animal experiments were performed using the methods of treatment of tumors using a recombinant oncolytic virus and/or a small-molecule anticancer drug provided in Preparation Examples 25, 42, 64, 81, 87, 114, 91 and 106. Details of the experiments are shown in Table 20.

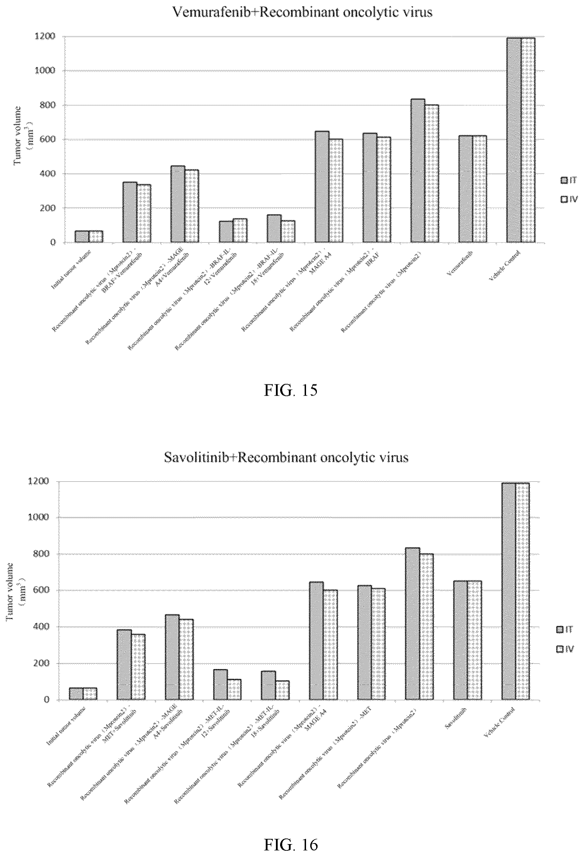

The test results are shown in FIG. 15.

**Table 20 Animal experiments on the small-molecule anticancer drug Vemurafenib**

| Group | Corresponding Preparation Example | Corresponding method | Number of animals | Dose | Administrat ion volume (mL/kg) | Administrat ion frequency | Administrat ion route | Administrat ion route |
|---|---|---|---|---|---|---|---|---|
| 1 | 25 | Recombinant oncolytic virus (M protein 2)-BRAF+Vemurafenib | 10 | 3e8 PFU/animal+ 150 mg/kg | 0.05+10 | Q2D*6 + QD*12 | IT+PO | IV+PO |
| 2 | 42 | Recombinant oncolytic virus (M protein 2)-MAGE A4+Vemurafenib | 10 | | | | | |
| 3 | 64 | Recombinant oncolytic virus (M protein 2)-BRAF-IL-12+Vemura fenib | 10 | | | | | |
| 4 | 81 | Recombinant oncolytic virus (M protein 2)-BRAF-IL-18+Vemura fenib | 10 | | | | | |
| 5 | 87 | Recombinant oncolytic virus (M protein 2)-MAGE A4 | 10 | | | | | |
| 6 | 114 | Recombinant oncolytic virus (M protein 2)-BRAF | 10 | | | | | |
| 7 | 91 | Recombinant oncolytic virus (M protein 2) | 10 | 3e8 PFU/animal | *0.05* | Q2D*6 | IT | IV |
| 8 | 92 | Vemurafenib | 10 | 150mg/kg | 10 | QD*12 | PO | PO |
| 9 | Vehicle Control | | 10 | N/A | 10 | QD*12 | PO | PO |

### 16. Animal experiments on the small-molecule anticancer drug Savolitinib

Animal experiments were performed using the methods of treatment of tumors using a recombinant oncolytic virus and/or a small-molecule anticancer drug provided in Preparation Examples 26, 43, 65, 82, 87, 115, 91 and 107. Details of the experiments are shown in Table 21.

The test results are shown in FIG. 16.

**Table 21 Animal experiments on the small-molecule anticancer drug Savolitinib**

| Gro up | Correspond ing Preparation Example | Corresponding method | Numb er of anima Is | Dose | Administra tion volume (mL/kg) | Administra tion frequency | Administra tion route | Administra tion route |
|---|---|---|---|---|---|---|---|---|
| 1 | 26 | Recombinant oncolytic virus (M protein 2)-MET+Savolitinib | 10 | 3e8 PFU/animal +90 mg/kg | 0.05+10 | Q2D*6 + QD*12 | IT+PO | IV+PO |
| 2 | 43 | Recombinant oncolytic virus (M protein 2)-MAGE A4+Savolitinib | 10 | | | | | |
| 3 | 65 | Recombinant oncolytic virus (M protein 2)-MET-IL-12+Savo litinib | 10 | | | | | |
| 4 | 82 | Recombinant oncolytic virus (M protein 2)-MET-IL-18+Savo litinib | 10 | | | | | |
| 5 | 87 | Recombinant oncolytic virus (M protein 2)-MAGE A4 | 10 | | | | | |
| 6 | 115 | Recombinant oncolytic virus (M protein 2)-MET | 10 | | | | | |
| 7 | 91 | Recombinant oncolytic virus (M protein 2) | 10 | 3e8 PFU/animal | 0.05 | Q2D*6 | IT | IV |
| 8 | 92 | Savolitinib | 10 | 90mg/kg | 10 | QD*12 | PO | PO |
| 9 | Vehicle Control | | 10 | N/A | 10 | QD*12 | PO | PO |

### 17. Animal experiments on the small-molecule anticancer drug Sotorasib

Animal experiments were performed using the methods of treatment of tumors using a recombinant oncolytic virus and/or a small-molecule anticancer drug provided in Preparation Examples 27, 44, 66, 83, 87, 116, 91 and 108. Details of the experiments are shown in Table 22.

The test results are shown in FIG. 17.

**Table 22 Animal experiments on the small-molecule anticancer drug Sotorasib**

| Group | Correspond ing Preparation Example | Corresponding method | Number of animals | Dose | Admini stration volume (mL/kg) | Adm inistr ation frequ ency | Adm inistr ation route | Adm inistr ation route |
|---|---|---|---|---|---|---|---|---|
| 1 | 26 | Recombinant oncolytic virus (M protein 2)-KRAS G12C+Sotorasib | 10 | 3e8 PFU/anim al+100 mg/kg | 0.05+1 0 | Q2D *6 + QD* 12 | IT+P O | IV+P O |
| 2 | 43 | Recombinant oncolytic virus (M protein 2)-MAGE A4+Sotorasib | 10 | | | | | |
| 3 | 65 | Recombinant oncolytic virus (M protein 2)-KRAS G12C-IL-12+Sotorasib | 10 | | | | | |
| 4 | 82 | Recombinant oncolytic virus (M protein 2)-KRAS G12C-IL-18+Sotorasib | 10 | | | | | |
| 5 | 87 | Recombinant oncolytic virus (M protein 2)-MAGE A4 | 10 | | | | | |
| 6 | 116 | Recombinant oncolytic virus (M protein 2)-KRAS G12C | 10 | | | | | |
| 7 | 91 | Recombinant oncolytic virus (M protein 2) | 10 | 3e8 PFU/anim al | 0.05 | Q2D *6 | IT | IV |
| 8 | 92 | Sotorasib | 10 | 100mg/kg | 10 | QD*12 | PO | PO |
| 9 | Vehicle Control | | 10 | N/A | 10 | QD* 12 | PO | PO |

The test results are shown in FIGS. 1-17.

Referring to the figures, the method for treatment of a tumor by using a recombinant oncolytic virus in combination with a small-molecule anticancer drug provided by the present application has obvious inhibitory effect on tumor growth. Moreover, the test results of using a recombinant oncolytic virus in direct combination with a small-molecule anticancer drug to treat tumors are superior to the test results of using the recombinant oncolytic virus or the small-molecule anticancer drug alone. The test results of using a recombinant oncolytic virus in combination with an antigen and then with a small-molecule anticancer drug to treat tumors are superior to the test results of using the recombinant oncolytic virus in direct combination with the small-molecule anticancer drug or the test results of directly using the recombinant oncolytic virus in combination with the antigen. Therefore, the method for treatment of a tumor by using a recombinant oncolytic virus in combination with a small-molecule anticancer drug provided by the present application further effectively improves the treatment effect on tumor cells.

Moreover, according to the method for treatment of a tumor by using a recombinant oncolytic virus in combination with a small-molecule anticancer drug in the present application, the test results of where the recombinant oncolytic virus has a target paired with the small-molecule anticancer drug are superior to the test results of where the recombinant oncolytic virus has no target paired with the small-molecule anticancer drug. Further, the test results of where the recombinant oncolytic virus has a target paired with the small molecule anticancer drug and the recombinant oncolytic virus expresses the cytokine are superior to the test results of where the recombinant oncolytic virus has a target paired with the small molecule anticancer drug, but the recombinant oncolytic virus does not express the cytokine.

In view of the test results listed above, the method for treatment of a tumor by using a recombinant oncolytic virus in combination with a small-molecule anticancer drug provided by the present application has good inhibitory effect on the growth of tumor cells. It can be learnt that the method for treatment of a tumor by using a recombinant oncolytic virus in combination with a small-molecule anticancer drug provided by the present application also has good inhibitory effect on other cancer cells, thus having broad clinical application prospects.

The specific examples are merely an explanation of the present application and not for limiting the present application. Those skilled in the art may make modifications, without creative contribution, to the examples as needed after reading this specification. Any of the modifications made within the scope of the claims of the present application shall be protected by the Patent Law.

## Claims

1. A method for treatment of a tumor by using a recombinant oncolytic virus in combination with a small-molecule anticancer drug, **characterized in that** the small-molecule anticancer drug and the recombinant oncolytic virus are used in combination to treat the tumor;
the small-molecule anticancer drug comprises a small-molecule anticancer drug targeting ALK, a small-molecule anticancer drug targeting BTK, a small-molecule anticancer drug targeting EGFR, a small-molecule anticancer drug targeting FGFR, a small-molecule anticancer drug targeting HER2, a small-molecule anticancer drug targeting Parp, a small-molecule anticancer drug targeting PI3K, a small-molecule anticancer drug targeting VEGFR, a small-molecule anticancer drug targeting CDK4/6, and a small-molecule anticancer drug targeting KRAS;
the recombinant oncolytic virus comprises an M protein, a G protein, an N protein, a P protein, and an L protein;
compared to an amino acid sequence shown in SEQ ID NO 1,
the M protein comprises any one or more site mutations of M51R, V221F and S226R; or
the M protein comprises any one or more site mutations of N32S, N49D, M51R, H54Y, V221F, V225I and S226R; or
the M protein comprises any one or more site mutations of N32S, N49D, M51R, H54Y, knockouting of bases encoding leucine at position 111, V221F, V225I and S226R; or
the M protein comprises any one or more site mutations of N32S, N49D, M51R, H54Y, L111A, V221F, V225I and S226R; or
the M protein comprises any one or more site mutations of G21E, N32S, N49D, M51R, H54Y, V221F, V225I and S226R; or
the M protein comprises any one or more site mutations of G21E, N32S, M33A, N49D, M51R, H54Y, V221F, V225I and S226R; or
the M protein comprises any one or more site mutations of G21E, N32S, M33A, N49D, M51R, H54Y, A133T, V221F, V225I and S226R; or
the M protein comprises any one or more site mutations of N32S, M33A, N49D, M51R, H54Y, V221F, V225I and S226R; or
the M protein comprises any one or more site mutations of N32S, M33A, N49D, M51R, H54Y, A133T, V221F, V225I and S226R; or
the M protein comprises any one or more site mutations of N32S, N49D, M51R, H54Y, A133T, V221F, V225I and S226R;
compared to an amino acid sequence shown in SEQ ID NO 12, the G protein comprises any one or more site mutations of V53I, A141V, D172Y, K217E, D232G, V331A, V371E, G436D, T438S, F453L, T471I, and Y487H;
compared to an amino acid sequence shown in SEQ ID NO 14, the N protein comprises any one or more site mutations of 114V, R155K and S353N;
compared to an amino acid sequence shown in SEQ ID NO 16, the P protein comprises any one or more site mutations of R50K, V76A, D99E, L126S, L140S, H151Y, I168M, K170E, Y189S, and N237D; and
compared to an amino acid sequence shown in SEQ ID NO 18, the L protein comprises any one or more site mutations of S87P and I487T.

2. The method for treatment of a tumor by using a recombinant oncolytic virus in combination with a small-molecule anticancer drug according to claim 1, **characterized in that** the oncolytic virus comprises one or more selected from a group consisting of rhabdovirus, poxvirus, herpes simplex virus, measles virus, Semliki Forest virus, poliovirus, reovirus, Seneca Valley virus, Echo-type enterovirus, coxsackievirus, Newcastle disease virus, and Maraba virus.

3. The method for treatment of a tumor by using a recombinant oncolytic virus in combination with a small-molecule anticancer drug according to claim 2, **characterized in that** the rhabdovirus comprises a vesicular stomatitis virus (VSV).

4. The method for treatment of a tumor by using a recombinant oncolytic virus in combination with a small-molecule anticancer drug according to claim 1, **characterized in that** the recombinant oncolytic virus further comprises an antigen encoded by a first exogenous gene.

5. The method for treatment of a tumor by using a recombinant oncolytic virus in combination with a small-molecule anticancer drug according to claim 1, **characterized in that** the antigen is selected from a hematological tumor antigen and a solid tumor antigen.

6. The method for treatment of a tumor by using a recombinant oncolytic virus in combination with a small-molecule anticancer drug according to claim 1, **characterized in that**:
the solid tumor antigen comprises but is not limited to 5T4, RORI, EGFR, FcγRI, FcγRIIa, FcγRIIb, CD28, CD137, CTLA-4, HER2, HER3, FAS, FAP, LGR5, C5aR1, A2AR, FGFR1, FGFR2, FGFR3, FGFR4, glucocorticoid-induced TNFR-related (GITR) protein, LTβR, TRAIL receptor 1, TRAIL receptor 2, prostate-specific membrane antigen (PSMA) protein, prostate stem cell antigen (PSCA) protein, tumor-related protein carbonic anhydrase IX (CAIX), EGFR1, EGFRvIII, ErbB3, folate receptor, ephrin receptor, PDGFRa, ErbB-2, CD2, CD40, CD74, CD80, CD86, CCAM5, CCAM6, p53, MET, HGFR, MAGE-A1, MAGE-A2, MAGE-A3, MAGE-A4, MAGE-A6, MAGE-A10, MAGE-A12, BACE, DAM-6, DAM-10, GAGE-1, GAGE-2, GAGE-8, GAGE-3, GAGE-4, GAGE-5, GAGE-6, GAGE-7B, NA88-A, NY-ESO-1, BRCA1, BRCA2, MART-1, MCIR, Gp100, PSA, PSM, tyrosinase, TRP-1, TRP-2, ART-4, CAMEL, Cyp-B, hTERT, hTRT, iCE, MUC2, P-cadherin, myostatin, Cripto, MUC5AC, PRAME, P15, RU1, RU2, SART-1, SART-3, AFP, β-catenin/m, caspase-8/m, CDK-4/m, ELF2M, GnT-V, G250, HSP70-2M, HST-2, KIAA0205, MUM-1, MUM-2, MUM-3, myosin/m, RAGE, SART-2, TRP-2/INT2, 707-AP, annexin II, CDC27/m, TPI/mbcr-abl, ETV6/AML, LDLR/FUT, Pml/RARa, TEL/AML1, CD28, CD137, CanAg, mesothelin (MSLN), DR5, PD-1, PD-L1, IGF-1R, CXCR4, neuropilin 1 (NRP-1), glypican, EphA2, B7-H3, B7-H4, gpA33, GPC3, SSTR2, GD2, VEGF-A, VEGFR-2, PDGFR-a, ANKL, RANKL, MSLN, EBV, TROP2, FOLR1, AXL, Claude 18.2, MUC1, TPBG, CEA, and EpCAM; and
the hematological tumor antigen comprises but is not limited to BCMA, CD4, CD5, CD7, CD10, FcγRIIIa, FcγRIIIb, CD19, CD20, CD22, CD23, CD30, CD33, CD34, CD37, CD38, CD44, CD47, CD56, CD70, CD117, CD123, CD138, CD174 , CLL-1, ROR1, NKG2DL1/2, IL1R3, FCRL5, GPRC5D, CLEC12A, WT1, FLT3, TLR8, SHP2, KAT6A/B, CSNK1A1, FLI1, IKZF1/3, PI3K, c-Kit , SLAMF3, SLAMF7, TCR B-chain, ITGB7, k-1gG, TACI, TRBCI, and LeY.

7. The method for treatment of a tumor by using a recombinant oncolytic virus in combination with a small-molecule anticancer drug according to claim 6, **characterized in that** the antigen is one or more selected from a group consisting of: CD19, CD22, BCMA, MUC1, NY-ESO-1, MAGE A4, MET, Claude 18.2, MSLN, EGFR, VEGFR2, HER2, TPBG, AFP, and MAGE-A10.

8. The method for treatment of a tumor by using a recombinant oncolytic virus in combination with a small-molecule anticancer drug according to claim 4, **characterized in that** the recombinant oncolytic virus expresses at least one or more the antigens.

9. The method for treatment of a tumor by using a recombinant oncolytic virus in combination with a small-molecule anticancer drug according to claim 1, **characterized in that** the small-molecule anticancer drug comprises:
a small-molecule anticancer drug targeting ALK, comprising but not limited to Alectinib, Brigatinib, Ceritinib, Crizobtinib, Entrectinib, Lorlatinib, and Ensartinib;
a small-molecule anticancer drug targeting BTK, comprising but not limited to Acalabrutinib, Ibrutinib, Zanubrutinib, and Orelabrutinib;
a small-molecule anticancer drug targeting EGFR, comprising but not limited to Afatinib, Dacomitinib, Erlotinib, Gefitinib, Icotinib, Lapatinib, Mobocertinib, Osimertinib, Rybrevant, Befotertinib, RezivertinibDasatinib, Brigatinib, Vandetanib, Almonetinib, and Furmonertinib;
a small-molecule anticancer drug targeting FGFR, comprising but not limited to Erdafitinib, Infigratinib, Pemazyre, Pemigatinib, Lenvatinib, Pazopanib, Anlotinib, and Ponatinib;
a small-molecule anticancer drug targeting HER2, comprising but not limited to Neratinib, Pyrotinib, Tucatinib, and Mobocertinib;
a small-molecule anticancer drug targeting Parp, comprising but not limited to Avapritinib, Fluzoparib, Niraparib, Olaparib, and Rucaparib;
a small-molecule anticancer drug targeting PI3K, comprising but not limited to duvelisib and linperlisib;
a small-molecule anticancer drug targeting VEGFR, comprising but not limited to Apatinib, Axitinib, Lenvatinib, Pazopanib, Regorafenib, Anlotinib, Cabozantinib, Sunitinib, Vandetanib, Ponatinib, Sorafenib, Donafenib, Surufatinib, and Fruquintinib;
a small-molecule anticancer drug targeting CDK4/6, comprising but not limited to Abemaciclib, Dalpiciclib, Palbociclib, and Ribociclib;
a small-molecule anticancer drug targeting KRAS, comprising but not limited to small-molecule anticancer drugs targeting KRAS G12A, KRAS G12C, KRAS G12D, KRAS G12R, KRAS G12V, KRAS G13D, KRAS Q61L and KRAS Q61H; and
a small-molecule anticancer drug targeting KRAS G12C, comprising but not limited to Sotorasib and Adagrasib.

10. The method for treatment of a tumor by using a recombinant oncolytic virus in combination with a small-molecule anticancer drug according to claim 1, **characterized in that** the small-molecule anticancer drug is one or more selected from a group consisting of:
a small molecule anticancer drug indicated for melanoma, comprising but not limited to Vemurafenib, Dabrafenib, Encorafenib, Trametinib, Binimetmib, and Cobimetinib;
a small molecule anticancer drug indicated for cytoma or sarcoma, comprising but not limited to Pexidartinib, tazemetostat, Pazopanib, and Anlotinib;
a small molecule anticancer drug indicated for leukemia, comprising but not limited to Imatinib, Bosutinib, Nilotinib, Gilteritinib, and Ivosidenib; and
a small-molecule anticancer drug indicated for Non Small Cell Lung Cancer (NSCLC), comprising but not limited to Sotorasib, Capmatinib, Tepotinib, Savolitinib, Pralsetinib, selpercatinib, Alectinib, Brigatinib, Ceritinib, Crizobtinib, Entrectinib, Lorlatinib, Afatinib, Dacomitinib, Erlotinib, Gefitinib, Icotinib, Mobocertinib, Osimertinib, Rybrevant, and Befotertinib.

11. The method for treatment of a tumor by using a recombinant oncolytic virus in combination with a small-molecule anticancer drug according to claim 1, **characterized in that** the small-molecule anticancer drug is one or more selected from a group consisting of: tivazanib, Everolimus, Sirolimus, Temsirolimus, Midostaurin, Ripretinib, Selumetinib, Larotrectinib, Lurbinectedin, and Olverembatinib.

12. The method for treatment of a tumor by using a recombinant oncolytic virus in combination with a small-molecule anticancer drug according to claim 1, **characterized in that** the small-molecule anticancer drug is one or more selected from a group consisting of:
a single-target small-molecule anticancer drug, comprising but not limited to: Lorlatinib, Acalabrutinib, Ibrutinib, Zanubrutinib, Erlotinib, Gefitinib, Icotinib, Osimertinib, Rybrevant, Befotertinib, Rezivertinib, Erdafitinib, Infigratinib, Pemazyre, Pemigatinib, Pyrotinib, Tucatinib, Avapritinib, Fluzoparib, Niraparib, Olaparib, Rucaparib, linperlisib, Apatinib, Abemaciclib, Dalpiciclib, Palbociclib, Ribociclib, Vemurafenib, Dabrafenib, Encorafenib, Trametinib, Binimetmib, Cobimetinib, Pexidartinib, tazemetostat, Gilteritinib, Ivosidenib, Sotorasib, Capmatinib, Tepotinib, Savolitinib, Pralsetinib, selpercatinib, Everolimus, Sirolimus, Temsirolimus, Midostaurin, Ripretinib, Selumetinib, Larotrectinib, and Lurbinectedin; and
a multi-target small-molecule anticancer drug, comprising but not limited to: Alectinib, Brigatinib, Ceritinib, Crizobtinib, Entrectinib, Afatinib, Dacomitinib, Lapatinib, Mobocertinib, Dasatinib, Neratinib, duvelisib, Axitinib, Lenvatinib, Pazopanib, Regorafenib, Anlotinib, Cabozantinib, Sunitinib, Vandetanib, Ponatinib, Sorafenib, Imatinib, Bosutinib, Nilotinib, and tivazanib.

13. The method for treatment of a tumor by using a recombinant oncolytic virus in combination with a small-molecule anticancer drug according to claim 1, **characterized in that** the small-molecule anticancer drug is one or more selected from a group consisting of: Ceritinib, Ibrutinib, Afatinib, Dacomitinib, Icotinib, Lenvatinib, Pazopanib, Anlotinib, Pyrotinib, Niraparib, Olaparib, Regorafenib, Palbociclib, Vemurafenib, Savolitinib, Everolimus, Mobocertinib, and Sotorasib.

14. The method for treatment of a tumor by using a recombinant oncolytic virus in combination with a small-molecule anticancer drug according to claim 1, **characterized in that** the recombinant oncolytic virus further comprises a cytokine encoded by a second exogenous gene.

15. The method for treatment of a tumor by using a recombinant oncolytic virus in combination with a small-molecule anticancer drug according to claim 14, **characterized in that** the cytokine is selected from interleukins (IL), interferons (IFN), tumor necrosis factors (TNF), colony stimulating factors (CSF), transforming growth factor-β family (TGF-β family) and chemokine family.

16. The method for treatment of a tumor by using a recombinant oncolytic virus in combination with a small-molecule anticancer drug according to claim 15, **characterized in that** the cytokine is one or more selected from a group consisting of: GM-CSF, G-CSF, M-CSF, IL-1, IL-2, IL-4, IL-5, IL-6, IL-9, IL-10, IL-12, IL-13, IL-15, IL-17, IL-18, IL-23, IL-27, IFN-a, IFN-β, IFN-γ, IFN-β, TGF-β, and TNF-α.

17. The method for treatment of a tumor by using a recombinant oncolytic virus in combination with a small-molecule anticancer drug according to claim 16, **characterized in that** the cytokine is one or more selected from a group consisting of: GM-CSF, IL-2, IL-12, IL-15, IL-18, IFN-β, and TNF-α.

18. The method for treatment of a tumor by using a recombinant oncolytic virus in combination with a small-molecule anticancer drug according to claim 1, **characterized in that** the recombinant oncolytic virus comprises a nucleic acid molecule; the nucleic acid molecule comprises a nucleic acid sequence encoding the M protein with the site mutation(s), a nucleic acid sequence encoding the G protein with the site mutation(s), a nucleic acid sequence encoding the N protein with the site mutation(s), a nucleic acid sequence encoding the P protein with the site mutation(s), and a nucleic acid sequence encoding the L protein with the site mutation(s).

19. The method for treatment of a tumor by using a recombinant oncolytic virus in combination with a small-molecule anticancer drug according to claim 18, **characterized in that** the nucleic acid molecule further comprises a nucleic acid sequence encoding the cytokine; and/or, the nucleic acid molecule further comprises a nucleic acid sequence encoding the antigen.

20. The method for treatment of a tumor by using a recombinant oncolytic virus in combination with a small-molecule anticancer drug according to claim 19, **characterized in that** the nucleic acid sequence encoding the cytokine is located between the nucleic acid sequence encoding the G protein with the site mutation(s) and the nucleic acid sequence encoding the L protein with the site mutation(s); and/ or, the nucleic acid sequence encoding the antigen is located between the nucleic acid sequence encoding the G protein with the site mutation(s) and the nucleic acid sequence encoding the L protein with the site mutation(s).

21. The method for treatment of a tumor by using a recombinant oncolytic virus in combination with a small-molecule anticancer drug according to claim 1, **characterized in that** the method for treatment of a tumor by using a recombinant oncolytic virus in combination with a small-molecule anticancer drug is used for killing an abnormally proliferative cell in a sustained manner.

22. The method for treatment of a tumor by using a recombinant oncolytic virus in combination with a small-molecule anticancer drug according to claim 21, **characterized in that** the abnormally proliferative cell is selected from tumor cells or tumor tissue-related cells.

23. The method for treatment of a tumor by using a recombinant oncolytic virus in combination with a small-molecule anticancer drug according to claim 21, **characterized in that** the tumor comprises solid tumors or hematological tumors.

24. The method for treatment of a tumor by using a recombinant oncolytic virus in combination with a small-molecule anticancer drug according to claim 21, **characterized in that** the tumor comprises but is not limited to acute lymphoblastic leukemia, acute B-cell leukemia, chronic non-lymphocytic leukemia, non-Hodgkin's lymphoma, anal cancer, astrocytoma, basal cell carcinoma, bile duct cancer, bladder cancer, mastocarcinoma, breast cancer, cervical cancer, chronic myeloproliferative tumors, colorectal cancer, endometrial cancer, ependymoma, esophageal cancer, diffuse large B-cell lymphoma, sensorineuroblastoma, Ewing's sarcoma, fallopian tube cancer, gallbladder cancer, gastric cancer, gastrointestinal carcinoid tumors, hepatocellular carcinoma, hypopharyngeal cancer, Kaposi sarcoma, kidney cancer, Langerhans cell hyperplasia, laryngeal cancer, liver cancer, lung cancer, melanoma, Merkel cell carcinoma, mesothelioma, oral cancer, neuroblastoma, non-small cell lung cancer, osteosarcoma, ovarian cancer, pancreatic cancer, pancreatic neuroendocrine tumors, pharyngeal cancer, pituitary tumors, prostate cancer, rectal cancer, renal cell carcinoma, retinoblastoma, skin cancer, small cell lung cancer, small intestine cancer, squamous neck cancer, testicular cancer, thymoma, thyroid cancer, uterine cancer, vaginal cancer and vascular tumors.

25. A composition, **characterized in that** the composition comprises the recombinant oncolytic virus and small-molecule anticancer drug according to claim 1.
